(19) Europäisches Patentamt
European Patent Office
Office européen des brevets

(11) EP 3 950 660 B1

(12) EUROPEAN PATENT SPECIFICATION

(45) Date of publication and mention of the grant of the patent:
**19.02.2025 Bulletin 2025/08**

(21) Application number: **19934375.7**

(22) Date of filing: **10.12.2019**

(51) International Patent Classification (IPC):
***C07C 68/065*** (2020.01) ***C07C 69/96*** (2006.01)
***B01J 31/02*** (2006.01) ***B01J 21/06*** (2006.01)
***B01J 21/08*** (2006.01) ***B01J 37/03*** (2006.01)

(52) Cooperative Patent Classification (CPC):
(C-Sets available)
**C07C 68/065; B01J 21/063; B01J 21/08; B01J 31/0282; B01J 31/0284; B01J 31/0295; B01J 37/036;** B01J 2231/49 (Cont.)

(86) International application number:
**PCT/CN2019/124323**

(87) International publication number:
**WO 2021/114092 (17.06.2021 Gazette 2021/24)**

(54) **METHOD FOR PREPARING DIMETHYL CARBONATE**

VERFAHREN ZUR HERSTELLUNG VON DIMETHYLCARBONAT

PROCÉDÉ DE PRÉPARATION DE CARBONATE DE DIMÉTHYLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2019 CN 201911253631**

(43) Date of publication of application:
**09.02.2022 Bulletin 2022/06**

(73) Proprietors:
- **Shenyang University of Chemical Technology Liaoning 110142 (CN)**
- **Shida Shinghwa Advanced Material Group Co., Ltd Dongying, Shandong 257503 (CN)**

(72) Inventors:
- **SHI, Lei Shenyang Liaoning 110142 (CN)**
- **GUO, Jianjun Shandong 257503 (CN)**
- **LI, Xin Shandong 257503 (CN)**
- **LIU, Mingyu Shenyang Liaoning 110142 (CN)**

(74) Representative: **Ter Meer Steinmeister & Partner Patentanwälte mbB Nymphenburger Straße 4 80335 München (DE)**

(56) References cited:
**CN-A- 101 735 064**
**CN-A- 103 467 435**
**CN-A- 103 521 263**
**CN-A- 103 641 720**
**CN-A- 103 641 720**
**CN-A- 109 704 968**
**CN-A- 109 704 968**
**CN-A- 109 704 968**

EP 3 950 660 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 68/065, C07C 69/96**

## Description

### TECHNICAL FIELD

The present disclosure belongs to chemistry and chemical engineering, solvents and polycarbonate industries, and particularly relates to a synthetic method of a new ionic liquid with strong alkalinity, an embedding-riveting and immobilized method of the new ionic liquid, and the use of prepared homogeneous and heterogeneous catalysts for the catalytic synthesis of dimethyl carbonate.

### BACKGROUND

Dimethyl carbonate (DMC) is an important organic synthesis intermediate, and could be used to synthesize polycarbonate, carbamate, isocyanate etc. DMC is nontoxic, comprises functional groups such as methyl, carbonyl and the like in its structure, and can replace virulent dimethyl sulfate and phosgene to carry out methylation reaction to synthesize a plurality of downstream products with high additional values. Therefore, DMC has wide application prospect in the fields such as plastics, dyes, food additives, medicine, pesticides etc. DMC also has a high dielectric constant and can be used as electrolyte for lithium ion battery. In addition, DMC has the advantages of high oxygen content, high octane number, good gasoline/water distribution coefficient, low toxicity, rapid biodegradation etc, and is considered as a potential fuel oil additive. Therefore, the synthesis of dimethyl carbonate is becoming more and more important in the fields of petroleum and chemical industry.

At present, the synthetic methods of DMC disclosed in literatures are mainly as follows: (1) phosgenation: the defects thereof include long operation period, high toxicity of phosgene, serious corrosion of byproduct (hydrogen chloride) to devices and high chlorine content of product; (2) methanol oxidative carbonylation: poor stability of the catalyst and many oxygen-containing byproducts; (3) direct synthesis from methanol and carbon dioxide [18]: usual DMC yield of lower than 20% limited by thermodynamics; (4) urea methanolysis: easy decomposition of urea to byproducts such as N-methylcarbamate and the like, and usual DMC yield of lower than 37% limited by thermodynamics; (5) transesterification: transesterification of Ethylene Carbonate (EC) or Propylene Carbonate (PC) and methanol (MeOH), which has achieved industrial production, lower price of raw materials, mild reaction condition, simple process flow and no corrosion to devices and thus is a promising production method.

Currently, in the industrial ten-thousand ton/year of transesterification of EC and MeOH, sodium methylate is used as catalyst, a molar ratio of EC to MeOH as raw materials is 1:6, and the catalyst is 0.7wt-1.0wt% of the mixed raw materials by weight. The mixed solution passes through atmospheric and reactive distillation column with the column bottom temperature of 72-76°C and the column top temperature of 63.6 °C, an azeotrope of DMC and MeOH is taken from the column top of the distillation column, and high-purity DMC is obtained through high-low pressure separation. However, this process has some problems: sodium methylate participates in the reaction, is sensitive to water and extremely easy to inactivate, and thus cannot be reused; and the strongly alkaline solid waste generated by the inactivation of sodium methylate is difficult to separate from homogeneous reaction system and pollutes the environment. Literatures further disclosed other catalysts such as homogeneous soluble strong alkali (such as sodium hydroxide or potassium hydroxide), organic ammonium salt, alkali metal carbonate, ionic liquid, but these catalysts also have the problems of rapid deactivation, incapability of being reused or less reused times and the like, thereby limiting a large-scale industrial application thereof. Therefore, it is necessary to develop a new catalyst with high activity and high stability for the transesterification of EC and MeOH.

The Ionic Liquids (ILs) have the advantages of friendly-environment, low saturated vapor pressure, good thermal stability and the like, has good activity in terms of the transesterification of ethylene carbonate and methanol, and is a homogeneous catalyst which is potential to replace the traditional catalyst. Ma Chengming et al disclosed that four kinds of ionic liquids with the same cation and different anions (i.e., 1-butyl-3-methylimidazolium bromide [BmIm]Br, 1-butyl-3-methylimidazolium tetrafluoroborate[BmIm]$BF_4$, 1-butyl-3-methylimidazolium bicarbonate [BmIm]$HCO_3$, and 1-butyl-3-methylimidazolium hydroxide [BmIm]OH) were used for transesterification of EC and MeOH, and found that [BmIm]OH was most effective, wherein when a molar ratio of methanol to ethylene carbonate was 10:1, the catalyst was 2% of the total raw material by weight, and the reaction was carried out at 67 °C for 4 h, EC conversion efficiency was 88.5%, and DMC selectivity was 90.3 % but the activity of the catalyst decreased to 76.9% after the catalyst was reused five times. Hye-Young Ju et al disclosed that under the condition that 0.89 wt% 1-ethyl-3-methylimidazolium chloride EMImCl was used as catalyst and the reaction was carried out at 160°C for 6 h, EC conversion efficiency was 80.4% and DMC selectivity was 97.6%. Zhenzhen Yang synthesized an ionic liquid with 1, 4-diazabicyclooctane as cation, and found that the catalytic activity and stability were comprehensively best when the anion is $OH^-$, and the EC conversion efficiency was 90%, the DMC yield was 81% when the reaction was carried out at 70°C for 6h with 1 mol% catalyst; and after the catalyst was reused four times, the catalytic activity decreased to 88%, and the yield decreased to 79%. Compared with sodium methylate (an equilibrium conversion efficiency was reached when the reaction was carried out for 5min), the above ionic

liquids have low activity and low DMC selectivity. And the inactivation phenomenon still exists for the disclosed ionic liquids during several reuses. The process for preparing dimethyl carbonate by catalytic distillation of ethylene carbonate and methanol needs to meet the requirements that an azeotrope of dimethyl carbonate and methanol is taken from the column top at 63.4-63.6°C, the temperature of the column bottom was kept at about 73-75°C, and it took 20-30 min for raw materials to fall into the column bottom from the column top through the catalytic distillation column. Therefore, a heterogeneous catalyst to be developed requires to exhibit high catalytic activity at 63-80°C, and a reaction equilibrium requires to be reached within 30 min. However, the activity of the heterogeneous catalyst disclosed by literatures and patents is significantly lower than the requirement of industrial application.

CN109704968A discloses a method for synthesizing dimethyl carbonate under the catalysis action of an ionic liquid. The bifunctional ionic liquid is adopted as a catalyst, and a transesterification reaction is performed between ethylene carbonate and methanol, wherein the use amount of the catalyst is 0.5-10 mol% of ethylene carbonate, the molar ratio of ethylene carbonate to methanol is 1:(5-30), the reaction temperature is 40-70 DEG C, and the reaction time is 2-12 h, so that dimethyl carbonate and ethylene glycol are synthesized with a high conversion rate and high selectivity. The method has the following advantages: (1) the bifunctional ionic liquid adopted as the catalyst has the effect of a homogeneous reaction and the characteristic of a heterogeneous catalyst, the shortcomings of conventional solid catalysts and the homogeneous catalysts are overcome, and high catalytic activity, easy separation, recyclability and no pollution to the environment are achieved; (2) since the bifunctional ionic liquid is applied to the reaction system, reactants can be activated under the synergistic action of anions and cations of the catalyst, higher catalytic activity is achieved, and synthesis operation of the catalyst is simple and easy.

CN103641720A discloses a method of synthesizing diethyl carbonate by a basic ionic liquid as a catalyst, belonging to the technical field of catalytic synthesis of carbonic ester by ionic liquids. According to the method, propylene carbonate and ethanol are taken as raw materials or ethylene carbonate and ethanol are taken as raw materials, and the basic ionic liquid is selected as the catalyst, so that diethyl carbonate is synthesized through ester exchange. Based on deficiencies of conventional catalysts (solid acids and alkalis, resin, alkali salt and the like) and advantages of the ionic liquid, the invention provides the method of synthesizing diethyl carbonate by the basic ionic liquid as the catalyst. The method has the advantages of high catalyst activity (selectivity is close to 100%, and yield is 45-65%), short synthetic route, mild reaction condition, less equipment corrosion, environment-friendliness, good stability of catalyst, easiness in separation and recovery, repeated use in maintaining high activity and the like.

## SUMMARY

According to one aspect of the present disclosure, a method for preparing dimethyl carbonate is provided. Aiming at the problems such as weak alkalinity and poor nucleophilicity of ionic liquids, poor thermal stability and easy inactivation of ionic liquids with strong alkalinity and the like disclosed in the literatures and patents, a new ionic liquid with strong alkalinity and having special structure and high temperature-resistant and high stability is developed, and a method for preparing a heterogeneous catalyst with ionic liquid embedded in a riveting manner based on the homogeneous ionic liquid is further developed. A series of developed ionic liquids with strong alkalinity are used for synthesizing dimethyl carbonate and ethylene glycol by transesterification between ethylene carbonate and methanol, and exhibit extremely high reaction activity. A reaction equilibrium could be reached when the reaction is carried out at a temperature ranging from 68°C to70°Cfor 5 min even with 0.3 wt% catalyst. The alkaline ionic liquids still exhibit certain catalytic activity even at 0°C.

The synthesized ionic liquids exhibit substantially unchanged catalytic activity and show higher stability, even after reused 20 times. The prepared ionic liquids with strong alkalinity directly replace soluble strong alkali such as sodium hydroxide or potassium hydroxide to facilitate hydrolysis of single solution of ethyl orthosilicate, tetrabutyl titanate or aluminum silicate and the like, or hydrolysis of any mixed solution of thereof. The ionic liquid is embedded, by means of inlay, in silica, titanium dioxide, aluminum oxide or single/multiple oxygen-containing compound framework(s) with specific structure(s), without introducing Na+ or K+ ions at all. The prepared heterogeneous catalyst does not need to be baked, and can be directly applied to transesterification after being dried/vacuum dried, and exhibits excellent catalytic activity. Because the ionic liquid matrix has a double-nitrogen ring structure and can be embedded in (like a double-headed mountaineering cone) the crystal lattice of -Si-O-, -Ti-O-, -Al-O-or oxygen-containing composite compound, the immobilized heterogeneous catalyst shows higher stability, and the active component of ionic liquid with strong alkalinity is not easy to lose.

A method for preparing dimethyl carbonate, characterized in that, the method comprises following steps: contacting a raw material comprising ethylene carbonate and methanol with a catalyst to produce the dimethyl carbonate; wherein the catalyst comprises an ionic liquid with an anion and a cation, wherein the anion and the cation both comprise a nitrogen-containing heterocycle, wherein the catalyst further comprises a support, and the ionic liquid is embedded in the support in a riveting manner.

Optionally, the cation has a structure represented by Formula I or Formula II;

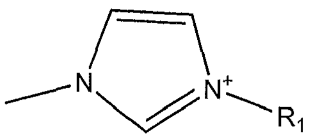 Formula I 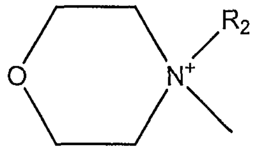 Formula II

the anion has a structure represented by Formula III, Formula IV or Formula V;

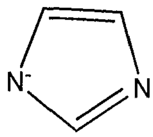 Formula III 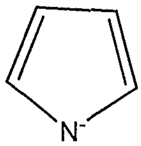 Formula IV 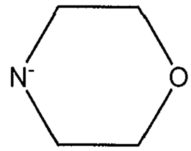 Formula V

wherein, $R_1$ and $R_2$ independently from each other are C1-C6 alkyl, C2-C6 alkenyl or C3-C6 aryl.

Optionally, the catalyst is in a range from 0.1% to 10% of the raw material by weight.

Optionally, the catalyst is in a range from 0.1% to5% of the raw material by weight.

Optionally, the catalyst is in a range from 0.1% to0.3% of the raw material by weight. Optionally, the reaction temperature is in a range from 60°C to 80°C.

Optionally, the reaction temperature is in a range from 66°C to 69°C.

Optionally, the reaction temperature is in a range from 76°C to 77°C.

Optionally, a molar ratio of ethylene carbonate to ethanol in the raw material comprising ethylene carbonate and methanol is in a range from 1: 1 to 1: 15.

Optionally, the molar ratio of ethylene carbonate to ethanol in the raw material comprising ethylene carbonate and methanol is in a range from 1: 4 to1: 15.

Optionally, the molar ratio of ethylene carbonate to ethanol in the raw material comprising ethylene carbonate and methanol is in a range from 1: 10 to 1: 15.

Optionally, the upper limit of the molar ratio of ethylene carbonate to ethanol in the raw material comprising ethylene carbonate and methanol is 1:2, 1:3, 1:4, 1:5, 1:6, 1:7, 1:8, 1:9, 1:10, 1:11, 1:12, 1:13, 1:14, or 1: 15, while the lower limit thereof is 1:1, 1:2, 1:3, 1:4, 1:5, 1:6, 1:7, 1:8, 1:9, 1:10, 1:11, 1:12, 1:13, or 1:14. Optionally, the reaction is carried out for a reaction time ranging from 1 minute to 30 h.

Optionally, a chemical equilibrium of the reaction is reached within 5 min when the reaction temperature is at 68°C.

Optionally, a chemical equilibrium is reached when the reaction time is 1 min and the reaction temperature is 68°C.

Optionally, $R_1$ and $R_2$ independently from each other are $-CH_3$, $-CH_2CH_3$, $-(CH_2)_2CH_3$, or $-(CH_2)_3CH_3$.

The catalyst further comprises a support, and the ionic liquid is embedded in the support in a riveting manner.

Optionally, the ionic liquid is embedded in the framework of the support.

Optionally, at least one action of coupling and hydrogen bonding exists between the support and the ionic liquid.

Optionally, a weight ratio of the ionic liquid to the support in the catalyst is in a range of 0.01~0.3: 0.1~0.6.

Optionally, the support is at least one selected from silica, titanium dioxide and aluminum oxide, and the ionic liquid is embedded in the framework of the support during the preparation of the support.

Optionally, the catalyst further comprises a structure regulator, and the ionic liquid is embedded in a composite framework of the structure regulator and the support. The structure regulator has a valence of 2, 3 or 4, and is used to construct a framework structure of composite oxide(s) together with the support, so as to improve the environment where the ionic liquid is embedded, thereby enhancing acting force between the ionic liquid and the composite oxide of support.

Optionally, the structure regulator comprises at least one selected from Mg, Ca, Ba, La, Ce, Zr, Fe, Zn, Li, Cs, and Al.

Optionally, the weight content of the structure regulator in the catalyst is in a range from 10% to 60%.

Optionally, the ionic liquid is prepared by a method comprising the following steps: a1) adding an alkali into solution I comprising an ionic liquid anion source, and reacting to obtain ionic liquid anion metal salt; and a2) dissolving the ionic liquid anion metal salt in a solvent, then adding an ionic liquid cation salt, and reacting to obtain the ionic liquid.

Optionally, in step a1), the solvent in solution I is at least one selected from ethanol, benzene, toluene and xylene; the alkali is an organic alkali or an inorganic alkali, wherein the organic alkali comprises sodium methylate, sodium ethylate or sodium tert-butoxide, potassium methylate, potassium ethylate or potassium tert-butoxide, and the inorganic alkali comprises sodium hydroxide or potassium hydroxide; and the ionic liquid anion metal salt is at least one selected from the ionic liquid anion sodium salts and the ionic liquid anion kalium salts.

Optionally, in step a1), the concentration of the ionic liquid anion source in the solution I is in a range from

0.05g/mL to 0.8 g/mL; and a molar ratio of the ionic liquid anion source to the alkali is in a range from 0.9 to 1.1.

Optionally, in step a1), the ionic liquid anion source comprises imidazole, pyrrole or morpholine.

Optionally, in step a1), reaction conditions are as follows: the reaction temperature is in a range from 50°C to 80°C and the reaction time is in a range from 5h to 12 h.

Optionally, step a1) further comprises: after the reaction is completed, removing the solvent in the reaction system to obtain imidazole anion salt, pyrrole anion salt or morpholine anion salt.

Optionally, in step a2), the solvent comprises a water-carrying agent which is at least one selected from ethanol, benzene, toluene and xylene; and the ionic liquid cation salt is at least one selected from 1-$R_1$-3-methyl-imidazolium bromide, 1-R1-3-methylimidazolium iodide, N-methyl-N-$R_2$-morpholinium bromide and N-methyl-N-$R_2$-morpholinium iodide.

Optionally, in step a2), the ratio of the ionic liquid anion metal salt to the solvent is in a range of 0.1~0.9: 0.05~1.2 g/mL.

Optionally, in step a2), reaction conditions are as follows: the reaction temperature is in a range from 25~80°C and the reaction time is in a range from12h to 48 h.

Optionally, step a2) further comprises: after the reaction is completed, removing the solvent in the reaction system to obtain the ionic liquid.

Optionally, a method for preparing the catalyst comprises step b): adding water into a mixture comprising a support precursor and the ionic liquid, and carrying out hydrolysis to obtain the catalyst.

Optionally, in step b), the support precursor comprises at least one selected from ethyl orthosilicate, tetrabutyl titanate, aluminum isopropoxide, and sodium aluminate.

Optionally, the mixture in step b) further comprises a solvent which is 10%~90% of the mixture by weight, and the solvent comprises at least one selected from methanol, ethanol, propanol, butanol, methyl acetate and ethyl acetate.

Optionally, in step b), a molar ratio of the support precursor, the ionic liquid and water is in a range of 0.2~0.8: 0.03~0.4: 0.1~0.4.

Optionally, in step b), the hydrolysis temperature is in a range from 25°C to 80°C.

Optionally, in step b), the upper limit of the temperature of the hydrolysis is 30°C, 35°C, 40°C, 50°C, 60°C or 70°C, while the lower limit thereof is 25°C, 30°C, 35°C, 40°C, 50°C or 60°C.

Optionally, in step b), after the hydrolysis, aging step is carried out to obtain the catalyst, wherein the aging temperature is in a range from 40°C to 120°C and the aging time is in a range from 6h to24 h.

Optionally, in step b), the mixture comprises a structure regulator precursor which comprises at least one selected from corresponding acetate, silicate, hydrochloride and nitrate of the structure regulator.

Optionally, step b) further comprises: mixing the support precursor, the structure regulator precursor with the solvent, adding the ionic liquid, mixing uniformly, and then adding water to carry out hydrolysis to obtain the catalyst.

Optionally, in step b), a weight ratio of the support precursor, the structure regulator precursor, the solvent, the ionic liquid and water is in a range of 0.2~0.8: 0.05~0.4: 0.3~0.8: 0.03~0.4: 0.1~0.4.

As an embodiment, the catalyst is a homogeneous ionic liquid with strong alkalinity.

A method for preparing the catalyst of homogeneous ionic liquid with strong alkalinity comprises the following steps:

(1) dissolving commercially available imidazole, pyrrole or morpholine in a certain amount of a solvent such as ethanol, benzene, toluene, xylene and the like; slowly adding equimolar organic or inorganic strong alkali into a three-neck flask, wherein the inorganic strong alkali comprises NaOH and KOH, and the organic strong alkali comprises sodium methoxide, sodium ethoxide, sodium tert-butoxide and the like, or potassium methoxide, potassium ethoxide, potassium tert-butoxide and the like; carrying out vigorous stirring, wherein the stirring temperature is in a range from 50 °C to 80°C and the stirring time is in a range from 5h to 12h; after the reaction is completed, carrying out rotary evaporation under the condition of reduced pressure and a temperature ranging from 60°C to 120°C in an oil bath to remove the solvent and alcohols or water produced; then drying in a vacuum drying oven until no weight change occured to obtain the imidazole salt, the pyrrole salt or the morpholine salt, wherein the obtained imidazole salt, the pyrrole salt or the morpholine salt has Na or K cation, namely the imidazole Na or K salt, the pyrrole Na or K salt and the morpholine Na or K salt;

(2) dissolving a certain amount of imidazole salt, pyrrole salt or morpholine salt having Na or K cation in a certain amount of water-carrying agent such as ethanol, benzene, toluene, xylene and the like, adding equimolar commercially available ionic liquid of 1-R-3-methylimidazolium bromide/iodide or N-methyl-N-R-morpholinium bromide/iodide into a three-neck flask to perform cation reaction, and carrying out vigorous stirring to react for a reaction time ranging from 12h to 48h to obtain NalKBr or Na/KI precipitate and target ionic liquid, wherein the stirring temperature is in a range from 25°C to 80°C; and

(3) filtering the mixture comprising the target ionic liquid many times, carrying out rotary evaporation under the condition of reduced pressure and a temperature ranging from 65°C to 100°C for a time ranging from 1h to4h in an oil

bath to remove the solvent, and drying in a vacuum drying oven until no weight change occurred to obtain a target product with pure components.

The imidazole-based, pyrrole-based or morpholine-based ionic liquid has the following structure, wherein the cation is 1-R-3-methylimidazolium or N-methyl-N-R-morpholinium, and R is a normal or isomeric structure of alkane, alkene or arene; and the anion is imidazole, pyrrole or morpholine anion.

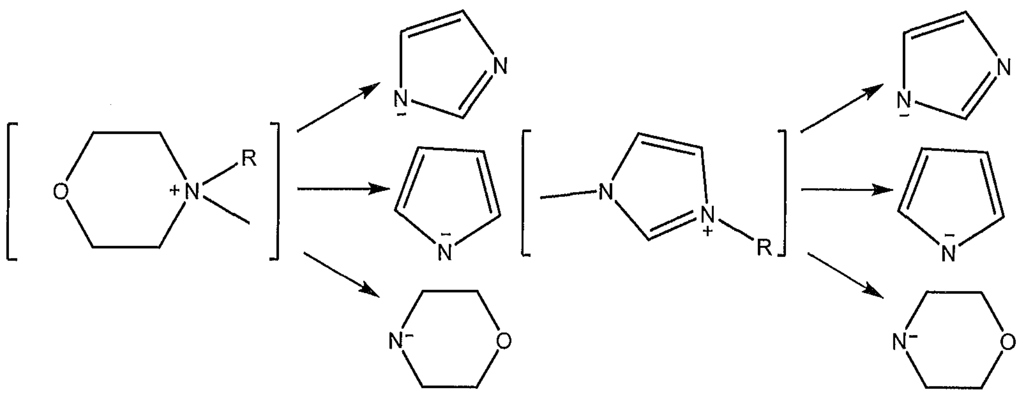

R = $-CH_3$, $-CH_2CH_3$, $-(CH_2)_2CH_3$, $-(CH_2)_3CH_3$ or other structure of alkane, alkene and arene

As an embodiment, the catalyst is a heterogeneous catalyst with the ionic liquid embedded in a riveting manner. In the method for preparing heterogeneous catalyst with the ionic liquid embedded in a riveting manner:

active components of the ionic liquid are alkalis or salts of imidazoles, pyrroles or morpholines, including but not limited to the ionic liquids such as 1-ethyl-3-methylimidazolium methoxide, 1-ethyl-3-methylimidazolium hydroxide, 1-ethyl-3-methylimidazolium imidazole salt, 1-ethyl-3-methylimidazolium pyrrole salt, 1-ethyl-3-methylimidazolium morpholine salt, 1-butyl-3-methylimidazolium imidazole salt, 1, 3-dimethylimidazolium pyrrole salt, N-methyl-N-ethylmorpholinium imidazole salt, N-methyl-N-ethylmorpholinium pyrrole salt, N-methyl-N-ethylmorpholinium morpholine salt, N-methyl-N-butylmorpholinium imidazole salt, and N-methyl-N-butylmorpholinium pyrrole salt etc;

the support precursor comprises but is not limited to one of ethyl orthosilicate, tetrabutyl titanate, aluminum isopropoxide, sodium aluminate and the like, or any combination thereof;

the structure regulator comprises one or more selected from Mg, Ca, Ba, La, Ce, Zr, Fe, Zn, Li, Cs and the like, which is/are added into the support precursor in the form of acetate, silicate or other organic metal salts;

the solvent comprises but is not limited to one of methanol, ethanol, propanol and butanol and the like of alcohols, or any combination thereof, or methyl acetate, ethyl acetate and the like of organic esters, or any combination thereof;

the support precursor, the structure regulator and the solvent are uniformly mixed, then a certain amount of single or multiple ionic liquids is added therein and uniformly mixed, then 0.8~1.5 times water of the calculated theoretical amount is added, and the temperature during hydrolysis is controlled to be in a range from *25°C* to 80°C to form a uniform gel product. The ionic liquid serves as both a promoter of hydrolysis and an active component in the final heterogeneous catalyst. After aging of the gel product at aging temperature from 40°C to 120°C for aging time in a range from 6h to24h, the solid product is rinsed from 3times to 5 times with the solvent of alcohols or esters to dissolve the ionic liquid attached to the surface thereof. Finally, vacuum drying is carried out at a temperature selected from 100°C to 150°C for drying time from 2h to 6h to obtain heterogeneous catalyst with strong alkalinity and having the ionic liquid embedded-riveted therein.

In the heterogeneous catalyst, the content of the active ionic liquid is in a range from 1% to30%, the content of the support is in a range from 10% to60%, and the content of the structure regulator/structure stabilizer is in a range from10% to60%.

In the present disclosure, "[EmIm]Im" refers to 1-ethyl-3-methylimidazolium imidazole salt.

In the present disclosure, "[EmIm]py" refers to 1-ethyl-3-methylimidazolyl pyrrole salt.

In the present disclosure, "EC" refers to ethylene carbonate.

In the present disclosure, "DMC" refers to dimethyl carbonate.

In the present disclosure, "EG" refers to ethylene glycol.

In the present disclosure, "HEMC", namely intermediate product 1, refers to "methanol glycol carbonate".

In the present disclosure, "intermediate product 2" refers to "diethylene glycol carbonate".

In the present disclosure, C1-C6 refers to the number of carbon atoms contained. For example, the term "C1-C6

alkyl" refers to alkyl containing 1-6 carbon atoms.

In the present disclosure, the "alkyl" is a group formed from an alkane compound molecule by losing any one of hydrogen atoms. The alkane compound comprises straight-chain alkanes, branched-chain alkanes, cycloalkanes and branched-chain cycloalkanes.

In the present disclosure, the "alkenyl" is a group formed from an olefin compound molecule by losing any one of hydrogen atoms.

In the present disclosure, an "aryl" is a group formed from an aromatic compound molecule by losing one hydrogen atom on an aromatic ring; for example, the p-tolyl is formed from toluene by losing the hydrogen atom at the para-position with respect to the methyl group on the phenyl ring.

The beneficial effects achieved by the present disclosure are as follows:

1) Aiming at the problems such as weak alkalinity, poor nucleophilicity of ionic liquids, poor thermal stability, easy inactivation of ionic liquids with strong alkalinity and the like disclosed in the literatures and patents, the present disclosure provides a method for preparing dimethyl carbonate, develops a new ionic liquid with strong alkalinity and having special structure and high temperature-resistant and high stability, and further develops a method for preparing a heterogeneous catalyst with ionic liquid embedded therein in a riveting manner based on the homogeneous ionic liquid;

2) During the method for preparing dimethyl carbonate provided by the present disclosure, the present disclosure develops a series of ionic liquids with strong alkalinity which are used for transesterification between ethylene carbonate and methanol to produce dimethyl carbonate and the ethylene glycol and exhibit extremely high reaction activity. A reaction equilibrium can be reached at a temperature ranging from 68°C to 70°C for 5 min even with 0.3 wt% catalyst, and the catalyst still exhibits certain catalytic activity even at 0°C;

3) During the method for preparing dimethyl carbonate provided by the present disclosure, the prepared ionic liquids with strong alkalinity directly replace soluble strong alkali such as sodium hydroxide or potassium hydroxide to facilitate hydrolysis of single solution of ethyl orthosilicate, tetrabutyl titanate or aluminum silicate and the like, or hydrolysis of any mixed solution thereof. The ionic liquid is embedded, by means of inlay, in silica, titanium dioxide, aluminum oxide or single/multiple oxygen-containing compound framework(s) with specific structure(s), without introducing $Na^+$ or $K^+$ ions at all. The prepared heterogeneous catalyst does not need to be baked, and can be directly applied to transesterification after being dried/vacuum dried, and exhibits excellent catalytic activity; and

4) During the method for preparing dimethyl carbonate provided by the present disclosure, because the ionic liquid matrix has a double-nitrogen ring structure and can be embedded into (like a double-headed mountaineering cone) the three-dimensional framework of -Si-O-, -Ti-O-, -Al-O-or oxygen-containing composite compound, the immobilized heterogeneous catalyst shows higher stability, and the active component of strongly alkaline ionic liquid is not easy to lose.

## BRIEF DESCRIPTION OF THE FIGURES

FIG.1 is 1HNMR spectrum (Hydrogen-1 Nuclear Magnetic Resonance Spectrum) of [EmIm]Im ionic liquid synthesized by Example 2.

FIG.2 is 13CNMR spectrum (Carbon-13 Nuclear Magnetic Resonance Spectrum) of [EmIm]Im ionic liquid synthesized by Example 2.

FIG.3 is 1HNMR spectrum of [EmIm]py ionic liquid synthesized by Example 2.

FIG.4 is 13CNMR spectrum of [EmIm]py ionic liquid synthesized by Example 2.

FIG. 5 shows the effect of different molar ratios of raw materials on the transesterification of EC and MeOH.

FIG.6 shows the effect of reaction temperature on the transesterification of EC and MeOH.

FIG.7 shows the effect of the reuse times of catalyst on the DMC yield.

FIG.8 shows an immobilized catalyst of 1-ethyl-3 methylimidazolium imidazole ionic liquid prepared by means of embedding.

FIG.9 shows infrared spectrum of ionic liquid catalyst immobilized by different methods.

Fig. 10 is SEM image of heterogeneous catalyst with immobilized ionic liquid (Fig.a shows a catalyst impregnated with 6g ionic liquid; Figs.b, c, and d show catalysts with 3g, 6g, and 12g ionic liquid embedded, respectively).

## DETAILED DESCRIPTION

The present disclosure will be described in detail below with reference to examples, but is not limited to these examples.

Unless otherwise specified, the raw material and catalyst in the examples of the present disclosure are commercially available.

In the examples of the present disclosure, 1HNMR analysis, 13CNMR analysis, infrared spectrum analysis, SEM analysis, and XRD analysis are all conventional operations, and those skilled in the art can operate according to the instructions of the instrument.

The conversion efficiency and selectivity in the examples of the present disclosure are calculated as follows.

In the examples of the present disclosure, the conversion efficiency and selectivity are calculated based on the molar number of carbon.

EC conversion efficiency = (the molar number of produced DMC + the molar number of produced intermediate product 1 + the molar number of produced intermediate product 2)/(the molar number of produced DMC + the molar number of produced intermediate product 1+ the molar number of produced intermediate product 2 + the molar number of unreacted EC).

Conversion efficiency of methanol is not involved in the examples.

DMC selectivity = (the molar number of produced DMC)/(the molar number of produced DMC+ the molar number of produced intermediate product 1 + the molar number of produced intermediate product 2).

EG selectivity = (the molar number of produced EG)/(the molar number of produced DMC + the molar number of produced intermediate product 1 + the molar number of produced intermediate product 2).

$$\text{DMC yield} = (\text{DMC conversion efficiency}) * (\text{DMC selectivity}) * 100\%.$$

### Example 1

A series of typical and commercialized ionic liquids were selected, and the following acidic ionic liquids were researched in terms of the effects on the transesterification of Ethylene Carbonate (EC) and methanol (MeOH): 1-ethyl-3-methylimidazolium ethyl sulfate ([Emim]$C_2H_5SO_4$), 1-ethyl-3-methylimidazolium hexafluoroantimonate ([Emim]$SbF_6$), 1-ethyl-3-methylimidazolium p-methylbenzenesulfonate([Emim] ToS), 1-butyl-3-methylimidazolium tetrafluoroborate ([Bmim]$BF_4$), 1-ethyl-3-methylimidazolium tetrafluoroborate ([Emim]$BF_4$), 1-butyl-3-methylimidazolium hexafluorophosphate ([Bmim]$PF_6$), 1-ethyl-3-methylimidazolium hexafluorophosphate ([Emim]$PF_6$), 1-butyl-3-methylimidazolium chloride ([Bmim]Cl), 1-ethyl-3-methylimidazolium chloride ([Emim]Cl), 1,3-dimethylimidazolium chloride ([Mmim]Cl), 1-butyl-3-methylimidazolium bromide ([Bmim]Br), 1-ethyl-3-methylimidazolium bromide ([Emim]Br), 1-butyl-3-methylimidazolium iodide ([Bmim]I) and 1-ethyl-3-methylimidazolium iodide ([Emim]I), 1,3-dimethylimidazolium iodide ([Mmim] I). The results were shown in table 1. The reaction conditions were as follows: EC: MeOH = 1:10, reaction temperature was 68°C, catalyst content was 3 wt%, and reaction time is 2 h.

TABLE 1

| Effects of commercialized ionic liquids on transesterification of EC and MeOH | | | | | |
|---|---|---|---|---|---|
| Catalyst | Conversion efficiency /% | Molar selectivity/% | | | Yield/ % |
| | EC | DMC | Intermediate product 1 | Intermediate product 2 | DMC |
| No catalyst | 10.13 | 0.87 | 98.63 | 0.00 | 0.09 |
| No catalyst[a] | 35.52 | 1.76 | 97.83 | 0.00 | 0.63 |

(continued)

| Effects of commercialized ionic liquids on transesterification of EC and MeOH | | | | | |
|---|---|---|---|---|---|
| Catalyst | Conversion efficiency /% | Molar selectivity/% | | | Yield/ % |
| | EC | DMC | Intermediate product 1 | Intermediate product 2 | DMC |
| [Emim]$C_2H_3SO_4$ | 0.00 | 0.00 | 0.00 | 0.00 | 0.00 |
| [Emim]$SbF_6$ | 0.00 | 0.00 | 0.00 | 0.00 | 0.00 |
| [Emim]ToS | 0.00 | 0.00 | 0.00 | 0.00 | 0.00 |
| [Bmim]$BF_4$ | 15.00 | 1.10 | 98.90 | 0.00 | 0.17 |
| [Emim]$BF_4$ | 20.00 | 1.11 | 98.89 | 0.00 | 0.22 |
| [Bmim]$PF_6$ | 27.93 | 1.00 | 98.35 | 0.00 | 0.28 |
| [Emim]$PF_6$ | 30.52 | 1.02 | 98.98 | 0.00 | 0.31 |
| [Bmim]Cl | 31.40 | 9.71 | 88.58 | 1.71 | 3.05 |
| [Emim] Cl | 34.67 | 35.92 | 62.27 | 1.81 | 12.45 |
| [Mmim]Cl | 40.39 | 49.78 | 48.77 | 1.45 | 20.11 |
| [Bmim]Br | 39.55 | 41.99 | 53.47 | 4.54 | 16.61 |
| [Emim]Br | 44.93 | 40.74 | 57.26 | 2.00 | 18.30 |
| [Bmim]I | 58.02 | 44.85 | 51.87 | 3.28 | 26.02 |
| [Emim]I | 58.08 | 55.79 | 42.40 | 1.81 | 32.40 |
| [Mmim]I | 61.91 | 67.47 | 31.06 | 1.47 | 41.77 |
| In the table 1, a refers to reaction time of 20 h. | | | | | |

As seen from table 1: the activities of the conventional and commercialized catalysts were not high, and under the condition that the optimal content of the ionic liquid [Mmim]I was 3% of the reaction raw material by weight and reaction time is 2 h , EC conversion efficiency of 61.91%, DMC selectivity of 67.47%, and DMC yield of only 41.77% were achieved.

**Example 2**

The specific preparation method of the 1-ethyl-3-methylimidazolium imidazole salt is as follow: 0.5mol imidazole was dissolved in 60 mL ethanol solvent, and equimolar potassium ethoxide was added into a three-neck flask, and they were vigorously stirred for 6 h at 60°C; after the reaction was completed, rotary evaporation under reduced pressure was carried out at 65°C in an oil bath for 2h to remove the solvent ethanol and the product ethanol, and the remains were placed in a vacuum drying oven and dried for 12h until no weight change occurred to obtain the imidazolium potassium salt; 0.5mol commercially available [EmIm]Br was added into a three-neck flask, and under the condition that a water-carrying agent ethanol was used as solvent, equimolar imidazolium potassium was added therein, and they were stirred and reacted at room temperature for 24h; after the reaction was completed, the resulting white solid KBr was removed by filteration, rotary evaporation under reduced pressure was carried out at 65°C in an oil bath for 2h to remove the solvent, and then the remains were placed in a vacuum drying oven and dried for 12h until no weight change occurred, to obtain viscous liquid with light yellow, i.e. [EmIm]Im, of which nuclear magnetic resonance spectrums are shown in FIGs.1 and 2. 1HNMR spectrum of synthesized [EmIm]Im ionic liquid in FIG. 1 shows the followings: [EmIm]Im 1HNMR (500 MHz, DMSO-d6) δ (ppm): 8.61 (d, J = 1.9 Hz, 1H, NCHN), 7.75 (d, J = 1.9 Hz, 1H, NCH), 7.58 (s, 1H, NCH), 6.23 (s, 2H, NCHCHN), 4.17 (q, *J* = 7.3 Hz, 2H, $NCH_2CH_3$), 3.83 (s, 3H, $NCH_3$), 1.37 (t, *J* = 7.3 Hz, 3H, $NCH_2CH_3$). 13CNMR spectrum of synthesized [EmIm] Im ionic liquid in FIG. 2 shows the followings: $^{13}$C NMR (126 MHz, DMSO-$d_6$) δ (ppm): 166.43 (s), 136.99 (s), 136.18 (s), 123.97 (s), 122.39 (s), 122.31 (s), 44.51 (s), 36.06 (s), 15.56 (s). Therefore, the synthesized ionic liquid was determined to be 1-ethyl-3-methylimidazolium imidazole salt.

The specific preparation method of the 1-ethyl-3-methylimidazolium pyrrole salt is as follow: 0.5mol pyrrole was dissolved in 60 mL ethanol solvent, equimolar potassium ethoxide was added into a three-neck flask, and they were vigorously stirred for 6 h at 70°C; after the reaction was completed, rotary evaporation under reduced pressure was carried out for 2h at 65°C in an oil bath to remove the ethanol solvent and the product ethanol, and the remains were placed into a vacuum drying oven and dried until no weight change occurred for 12 h to obtain the pyrrole potassium; 0.5mol

commercially available [EmIm]Br was added into a three-necked flask, and equimolar pyrrole potassium was added therein, and under the condition that a water-carrying agent ethanol is used as solvent, they were stirred at room temperature for 24 h; after the reaction was completed, the resulting white solid KBr was removed by filtration, rotary evaporation under reduced pressure was carried out at 65°C in an oil bath for 2h to remove the solvent, and the remains were placed in a vacuum drying oven and dried for 12h until no weight change occurred, to obtain viscous liquid with light yellow [EmIm]py, of which nuclear magnetic resonance spectrums are shown in FIGs.3 and 4. 1HNMR spectrum of synthesized [EmIm]py ionic liquid in FIG. 3 shows the followings: [EmIm]Py$^1$H NMR (500 MHz, DMSO-$d_6$) δ (ppm): 9.30 (s, 1H, NCHN), 7.84 (s, 1H, NCHCHN), 7.74 (s, 1H, NCHCHN), 6.74 (d, $J$ = 1.9 Hz, 2H, NCHCH), 6.02 (d, $J$ = 1.5 Hz, 2H, CHCHCH), 4.20 (q, $J$ = 7.3 Hz, 2H, $NCH_2CH_3$), 3.85 (s, 3H, $NCH_3$), 1.39 (t, $J$=7.3 Hz, 3H, $NCH_2CH_3$). 13CNMR spectrum of synthesized [EmIm]py ionic liquid in FIG. 4 shows the followings: $^{13}$C NMR (126 MHz, DMSO-$d_6$) δ (ppm) 136.71 (s), 123.96 (s), 122.41 (s), 117.79 (s), 107.56 (s), 44.56 (s), 36.20 (s), 15.63 (s). Therefore, the synthesized ionic liquid was determined to be 1-ethyl-3-methylimidazolium pyrrole salt.

**Example 3**

Table 2 shows the data of the synthesized ionic liquid in the laboratory for transesterification of EC and MeOH, and the preparation method of the ionic liquid in table 2 is the same as that of 1-ethyl-3-methylimidazolium imidazole salt in example 2, except that corresponding raw materials were changed. The evaluation conditions of the catalyst were as follows: a reaction temperature is 68°C, a reaction time is 5 min, a molar ratio of the raw material is EC:MeOH =1:10, and the catalyst is 0.3% of the raw material by weight.

TABLE 2

| Effects of the synthesized Ionic liquids on the activity of transesterification of EC and MeOH | | | | |
|---|---|---|---|---|
| Catalyst | EC Conversion efficiency(%) | DMC selectivity(%) | EG selectivity (%) | DMC yield (%) |
| 1-ethyl-3-methylimidazolium methoxide | 43.39 | 100 | 100 | 43.39 |
| 1-ethyl-3-methylimidazolium hydroxide | 80.50 | 100 | 100 | 80.50 |
| 1-ethyl-3-methylimidazolium imidazole salt | 89.93 | 100 | 100 | 89.93 |
| 1-ethyl-3-methylimidazolium pyrrole salt | 90.12 | 100 | 100 | 90.12 |
| 1-ethyl-3-methylimidazolium morpholine salt | 89.16 | 100 | 100 | 89.16 |
| 1-butyl-3-methylimidazolium imidazole salt | 88.63 | 100 | 100 | 88.63 |
| 1,3-dimethylimidazolium pyrrole salt | 90.28 | 100 | 100 | 90.28 |
| N-methyl-N-ethylmorpholinium imidazole salt | 88.45 | 100 | 100 | 88.45 |
| N-methyl-N-ethylmorpholinium pyrrole salt | 88.87 | 100 | 100 | 88.87 |
| N-methyl-N-ethylmorpholinium morpholine salt | 88.12 | 100 | 100 | 88.12 |
| N-methyl-N-butylmorpholinium imidazole salt | 88.34 | 100 | 100 | 88.34 |
| N-methyl-N-butylmorpholinium pyrrole salt | 88.48 | 100 | 100 | 88.48 |

The reaction conditions were as follows: a mole ratio of EC to MeOH in the raw material was 1:10, the amount of the catalyst was 0.3 wt%, and the reaction temperature was 68°C.

As seen from table 2, it is clear that the ionic liquid with strong alkalinity exhibits excellent catalytic activity, DMC and EG selectivity reaches 100%, and expect for 1-ethyl-3-methylimidazolium methoxide and 1-ethyl-3-methylimidazolium hydroxide, all the rest ionic liquids approached equilibrium conversion efficiency of about 89% under the reaction conditions.

**Example 4**

1,3-dimethylimidazolium pyrrole was used as catalyst, the reaction temperature was 68°C, the catalyst is 0.3% of the total raw materials by weight, a molar ratio of EC to MeOH (methanol) was 1:1, 1:4, 1:6, 1:10 and 1:15 respectively, and the reaction time was 5min. The effects of different molar ratios of raw material on the transesterification reaction of EC and MeOH are shown in FIG. 5. The reaction mechanism of EC and MeOH is as follow: Firstly, ring opening reaction of EC and

MeOH generated an intermediate HEMC, and transesterification of HEMC and MeOH generated the target DMC. However, when strongly alkaline catalyst 1, 3-dimethylimidazolium pyrrole salt was used, even if the MeOH content was very low (EC: MeOH = 1: 1) and the DMC yield was only 9.63%, the formation of the intermediate product HEMC and other byproducts were still not detected, indicating that the EC ring opening was the rate controlling step during the transesterification of EC and MeOH. At reaction temperature, it was not limited by thermodynamic equilibrium that transesterification of HEMC (intermediate 1, methanol glycol carbonate) and MeOH generated DMC. DMC yield increased rapidly to 63.33% when EC: MeOH = 1:4, and DMC yield continued increasing as MeOH content increased, and DMC yield increased to 90.28% when EC: MeOH =1:10, and DMC yield increased with small extent to 92.81% when a molar ratio of MeOH to EC was 1:15. The transesterification of EC and MeOH is a reversible process, and the conversion of EC could be promoted by increasing the proportion of raw material MeOH, so that the reaction could continue in the positive reaction direction, and thus the DMC yield could increase.

**Example 5**

N-methyl-N-ethylmorpholinium morpholine salt was used as catalyst, which is 0.3% of the total raw materials by weight. The DMC selectivity and DMC yield vary with temperature and reaction time, which are shown in FIG. 6. The DMC yield was 13.87 % when the reaction was carried out at 0°C for 5 min. Although the EC conversion efficiency was low at the time, the DMC selectivity was still higher than 80%. When the reaction was carried out at 0°C for 120 min, the EC conversion efficiency increased to 40.02%, the DMC selectivity was 100%, and the DMC yield was 40.02%, indicating that N-methyl-N-ethylmorpholinium morpholine salt still had a relatively strong ability of promoting transesterification of HEMC and MeOH even at low temperature. The yield was basically the same as that of [Mmim]I (the ionic liquid was 3% of the total raw material by weight) which had the best effects among the commercialized ionic liquids. The above data fully show that N-methyl-N-ethylmorpholinium morpholine salt ionic liquid still exhibits relatively high transesterification activity even at 0°C. As the reaction temperature gradually increased, the DMC yield increased and the DMC selectivity had been maintained as 100%, without generating any intermediate product. The DMC yield could reach 57.04% when the reaction was carried out at 50°C for 5 min. However, DMC yield reached 87.24% when the reaction was carried out at 68°C (reflux temperature) for 1 min, and DMC yield was basically the same as that for 1 min when the reaction was continued to 5 min, indicating that a chemical equilibrium was basically reached when the reaction was carried out at the reflux temperature for 1 min and the activity of N-methyl-N-ethylmorpholinium morpholine ionic liquid was extremely high, the product selectivity was 100%, and no intermediate product and byproduct were generated at all.

**Example 6**

1-ethyl-3-methylimidazolium hydroxide and N-methyl-N- ethylmorpholinium pyrrole salt were respectively used as catalysts to investigate their stabilities. Under the condition of 0.3 wt% ionic liquid, EC:MeOH (molar ratio) = 1:10, the reaction was carried out at 68°C and ended after 5 min. The resulting mixture was sampled and analyzed. Then rotary evaporation under reduced pressure -0.1 MPa was carried out at 150-155°C for 120 min, and the remaining ionic liquid was recycled for next cycle. FIG. 7 shows the relationship between the DMC yield and the reuse times of 1-ethyl-3-methylimidazolium hydroxide (i.e, "1-ethyl-3-methylcholine" in FIG. 7) ionic liquid and N-methyl-N-ethylmorpholinium pyrrole ionic liquid. The DMC yield was 80.50% when the 1-ethyl-3-methylimidazolium alkali was used for the first time, but the DMC yield decreased to 25.24% when the catalyst was reused once, which was only about a quarter of the initial activity. DMC yield continued decreasing as the reuse number increased, and the DMC yield was only 10.77% when the catalyst was reused four times. The above results fully show that [Emim]OH has poor stability, can only be used once, and deactivates quickly. In contrast, the synthesized N-methyl-N-ethylmorpholinium pyrrole ionic liquid was reused 20 times, the DMC yield maintained at 88%-90%, the DMC selectivity was 100%, no deactivation occurred, and excellent stability was exhibited with only 0.3wt% thereof. It can be seen that N-methyl-N-ethylmorpholinium pyrrole has not only good low-temperature catalytic activity, product selectivity of 100%, but also excellent thermal stability and reuse performance.

**Example 7**

A single silica obtained by hydrolyzing ethyl orthosilicate was used as support, and 1-ethyl-3 methylimidazolium imidazole ionic liquid is embedded therein in a riveting manner to prepare a heterogeneous catalyst with silica. 30mL ethyl orthosilicate and 21mL ethanol were added to a conical flask respectively, when they were heated to 60°C, about 12mL 1-ethyl-3methylimidazolium imidazole ionic liquid was immediately added to them, and after even mixing thereof, 8.9g deionized water was added therein. Among them, 1-ethyl-3 methylimidazolium imidazole ionic liquid not only plays a role of alkali catalysis to facilitate the hydrolysis of ethyl orthosilicate, but also serves as active center of transesterification reaction. After adding deionized water, the system gelled quickly. The gelled system was aged at 60°C for 12 h, then dried at 120°C for 10h, washed three times with 50 mL ethanol, and vacuum dried at 150°C for 3h, to obtain an immobilized ionic

liquid catalyst. The physical photo of the catalyst prepared in embedding-riveting manner was shown in FIG. 8. As seen from the physical photo, the catalyst prepared in embedding-riveting manner has apparent color of light yellow and has a crunchy texture.

**Example 8**

A fixed-bed reactor was used to evaluate the prepared heterogeneous catalyst. Reaction conditions were as follows: a molar ratio of ethylene carbonate to methanol in the reaction raw materials was 1:6, the reaction temperature was 76-77°C, the filling weight of catalyst was 4 g, and weight hourly space velocity of the reaction raw materials was 1 $h^{-1}$, that is, feeding weight per hour for the raw material was 4g, and the continuous evaluation time for each catalyst was not more than 1800 min. Table 3 shows the activity of the catalyst that was obtained by embedding 12g 1-ethyl-3 methylimidazolium imidazole ionic liquid at 50-70°C. The catalysts with $SiO_2$ as embedding support, which were prepared by hydrolysis at different temperatures, all exhibited DMC selectivity higher than 90%, but EC conversion efficiency of the reaction raw materials had significant differences. When the catalyst was hydrolyzed at 60°C, EC conversion efficiency was up to 62.88%. When the catalyst was hydrolyzed at 70°C, EC conversion efficiency was only 15.80%, which was very low. When the catalyst was hydrolyzed at 50 °C, EC conversion efficiency was 42.01%,which was moderate.

Table 3

| Effects of immobilized ionic liquid catalysts at different hydrolysis temperatures on the reaction of EC and MeOH | | | | |
|---|---|---|---|---|
| Conditions for preparing catalysts | EC Conversion efficiency (%) | intermediate product 1 selectivity (%) | intennediate product 2 selectivity (%) | DMC selectivity (%) |
| hydrolysis at 50°C | 42.01 | 6.84 | 1.05 | 90.15 |
| hydrolysis at 60°C | 62.88 | 4.86 | 0.77 | 94.13 |
| hydrolysis at 70°C | 15.80 | 8.71 | 0.84 | 91.89 |
| EC/MeOH=1/6, the embedding weight was 12g, reaction temperature was 76-77°C, the filling weight of the catalyst was 4g, a weight hourly space velocity was 1$h^{-1}$, and reaction time was 1800min. | | | | |

**Example 9**

Under a single silica obtained by hydrolyzing ethyl orthosilicate was used as support and hydrolysis was carried out at 60°C, 12g 1-ethyl-3-methylimidazolium pyrrole salt, 12g 1-ethyl-3-methyl imidazolium morpholine salt, 12g 1-butyl-3-methylimidazolium imidazole salt, 12g 1,3-dimethylimidazolium pyrrole salt, 12g N-methyl-N-ethylmorpholinium morpholine salt and 12g N-methyl-N-butylmorpholinium pyrrole salt were embedded therein, respectively. A fixed-bed reactor was used to evaluate the prepared heterogeneous catalyst. Reaction conditions were as follows: a molar ratio of ethylene carbonate to methanol in the reaction raw material was 1:6, the reaction temperature was 76-77°C, the filling weight of the catalyst was 4 g, and weight hourly space velocity of the reaction raw materials was 1 $h^{-1}$, that is, the feeding weight per hour for the raw material was 4g, and the continuous evaluation time for each catalyst was not more than 1800 min. Table 4 shows the activities of catalysts with different ionic liquids embedded.

Table 4

| Effects of catalysts with different ionic liquids embedded on the reaction of EC and MeOH | | | | |
|---|---|---|---|---|
| different ionic liquids embedded | EC conversion efficiency (%) | intermediate product 1 selectivity (%) | intermediate product 2 selectivity (%) | DMC selectivity (%) |
| 1-ethyl-3-methylimidazolium pyrrole salt | 63.01 | 4.82 | 0.68 | 94.50 |
| 1-ethyl-3-methyl imidazolium morpholine salt | 62.25 | 4.91 | 0.79 | 94.30 |
| 1-butyl-3-methylimidazolium imidazole salt | 61.59 | 5.02 | 0.84 | 94.14 |
| 1,3-dimethylimidazolium pyrrole salt | 63.35 | 4.07 | 0.59 | 95.34 |
| N-methyl-N-ethylmorpholinium morpholine salt | 60.99 | 5.89 | 0.83 | 93.28 |

(continued)

| Effects of catalysts with different ionic liquids embedded on the reaction of EC and MeOH | | | | |
|---|---|---|---|---|
| different ionic liquids embedded | EC conversion efficiency (%) | intermediate product 1 selectivity (%) | intermediate product 2 selectivity (%) | DMC selectivity (%) |
| N-methyl-N-butylmorpholinium pyrrole salt | 61.23 | 5.47 | 0.88 | 93.65 |
| EC/MeOH=1/6, the embedding weight was 12g, reaction temperature was 76-77°C, the filling weight of the catalyst was 4g, a weight hourly space velocity was $1h^{-1}$, and reaction time was 1800min. | | | | |

A single silica obtained by hydrolyzing ethyl orthosilicate was used as support, and different ionic liquids were embedded therein to obtain the catalysts. The catalysts all exhibited excellent capability in the transesterification of EC and MeOH, with EC conversion efficiency of above 60% and DMC selectivity of above 93%. Among them, 1,3-dimethylimidazolium pyrrole salt showed the best catalytic efficiency, with EC conversion efficiency of 63.55% and DMC selectivity of 95.34%.

### Example 10

Under a single silica obtained by hydrolyzing ethyl orthosilicate was used as support and 1,3-dimethylimidazolium pyrrole salt was used as active component, the effect of the prepared catalyst with reduced amount of the active component on the transesterification efficiency was studied. The catalyst was prepared as follow: 30 mL ethyl orthosilicate and 21mL ethanol were added into a conical flask, when they were heated to 60°C, 6g 1,3-dimethylimidazolium pyrrole salt ionic liquid was immediately added therein, after even mixing, 8.9g deionized water was added therein, and the system gelled quickly; the gelled system was aged at 60°C for 12 h, and dried at 120°C for 10 h, then washed three times with 50 mL ethanol, and vacuum dried at 150°C for 3 h to obtain an immobilized catalyst.

Table 5

| The effects of the catalysts with different embedding weights of 1,3-dimethylimidazolium pyrrole salt on the reaction of EC and MeOH | | | | |
|---|---|---|---|---|
| embedding weight of the active component | EC conversion efficiency (%) | intermediate product 1 selectivity (%) | intermediate product 2 selectivity (%) | DMC selectivity (%) |
| 12 g (as a comparison) | 63.35 | 4.07 | 0.59 | 95.34 |
| 6 g | 51.55 | 7.36 | 0.77 | 91.87 |
| EC/MeOH=1/6, the embedding weight of 1,3-dimethylimidazolium pyrrole salt was 6g, reaction temperature was 76-77°C, the filling weight of the catalyst was 4g, weight hourly space velocity was $1h^{-1}$, and reaction time was 1800min.. | | | | |

Under the single silica obtained by hydrolyzing ethyl orthosilicate was used as support and even 6g 1,3-dimethylimidazolium pyrrole salt ionic liquid was embedded therein, the catalyst obtained showed EC conversion efficiency of 51.55% and DMC selectivity of 91.87%.

### Example 11

Under titanium dioxide obtained by hydrolyzing tetrabutyl titanate was used as support and hydrolysis was carried out at 60°C, 12g 1-ethyl-3-methylimidazolium pyrrole salt, 12g 1-ethyl-3-methylimidazolium morpholine salt, 12g 1-butyl-3-methylimidazolium imidazole salt, 12g 1,3-dimethylimidazolium pyrrole salt, 12g N-methyl-N-ethylmorpholinium morpholine salt and 12g N-methyl-N-butylmorpholinium pyrrole salt were embedded in an embedding-riveting manner, respectively. A fixed-bed reactor was used to evaluate the prepared heterogeneous catalyst. Reaction conditions were as follows: a molar ratio of ethylene carbonate to methanol in the reaction raw material was 1:6, the reaction temperature was 76-77°C, the filling weight of the catalyst was 4g, weight hourly space velocity of the raw material was 1 $h^{-1}$, that is, feeding weight per hour for the raw material was 4g, and the continuous evaluation time for each catalyst was not more than 1800min. Table 6 shows the activities of catalysts with different ionic liquids embedded.

Table 6

| Effect of the catalysts with different ionic liquids embedded on the reaction of EC and MeOH | | | | |
|---|---|---|---|---|
| different ionic liquids embedded | EC conversion efficiency (%) | intermediate product1 selectivity (%) | intermediate product 2 selectivity (%) | DMC selectivity (%) |
| 1-ethyl-3-methylimidazolium pyrrole salt | 44.18 | 62.50 | 1.94 | 35.56 |
| 1-ethyl-3-methylimidazolium morpholine salt | 43.42 | 63.35 | 1.88 | 34.77 |
| 1-butyl-3-methylimidazolium imidazole salt | 42.99 | 62.89 | 1.28 | 35.83 |
| 1,3-dimethylimidazolium pyrrole salt | 48.18 | 50.69 | 0.88 | 48.43 |
| N-methyl-N-ethylmorpholinium morpholine salt | 40.76 | 68.23 | 2.18 | 29.59 |
| N-methyl-N-butylmorpholinium pyrrole salt | 42.13 | 63.55 | 1.36 | 35.09 |
| EC/MeOH=1/6, the embedding weight was 12g, reaction temperature was 76-77 °C, the filling weight of the catalyst was 4g, weight hourly space velocity of $1h^{-1}$, and reaction time was 1800min. | | | | |

Under different ionic liquids were embedded-riveted in titanium dioxide as support obtained by hydrolyzing tetrabutyl titanate, the obtained catalysts exhibited significantly lower catalytic activity than those with different ionic liquids embedded-riveted in silica as support, with EC conversion efficiency of 40%-45%, and DMC selectivity (except for 1,3-dimethylimidazolium pyrrole salt) less than 40%.

**Example 12**

An oxide obtained by hydrolyzing ethyl orthosilicate and tetrabutyl titanate was used as support, and 1-ethyl-3-methylimidazolium pyrrole salt ionic liquid was embedded therein. 14.5mL ethyl orthosilicate and 25mL ethanol were added to a conical flask, 23mL tetrabutyl titanate was added therein, then the structure regulator (5.08g zinc acetate, 4.96g magnesium acetate and 1.89g sodium aluminate) was added therein, and after even mixing, 9.35g deionized water was added. The resulting mixture was aged at 60°C for 12h, dried at 120°C for 10h, then washed three times with 50 mL ethanol, and vacuum dried at 150°C for 3h to obtain immobilized ionic liquid catalyst. A fixed-bed reactor was used to evaluate the prepared heterogeneous catalyst. Reaction conditions were as follows: a molar ratio of ethylene carbonate to methanol in the reaction raw material was 1:6, the reaction temperature was 76-77°C, the filling weight of the catalyst was 4 g, and weight hourly space velocity of the reaction raw material is $1h^{-1}$, that is, feeding weight for the raw material was 4g, and the continuous evaluation time for each catalyst was not more than 1800 min. The EC conversion efficiency was 56.78%, and the DMC selectivity was 62.39%, significantly higher ethylene carbonate transesterification efficiency than those of various catalysts with ionic liquids embedded in tetrabutyl titanate alone, and the DMC yield is 2.25 times higher than those of the catalysts with ionic liquids embedded in tetrabutyl titanate alone.

**Example 13**

Figure 9 showed infrared spectrums of the catalysts obtained by impregnating silica as support with 6g 1-ethyl-3-methyl-imidazolium imidazole salt and the catalysts obtained by embedding silica as support with 3g, 6g and 12g 1-ethyl-3-methyl-imidazolium imidazole salt, respectively. The strong absorption peak at $1064cm^{-1}$ was the stretching vibration characteristic peak of Si-O-Si, the absorption peak at $3427cm^{-1}$ was the stretching vibration peak of alcoholic O-H group, the absorption peak at $1631cm^{-1}$ was the characteristic absorption peak of C=C on the imidazole ring, and the characteristic absorption peak of the imidazole ring was at $797cm^{-1}$. It showed that the developed method for preparing immobilized ionic liquid catalyst in an embedding manner was reliable and feasible, and the active component ionic liquid was determined to be embedded in the heterogeneous catalyst. The height of the characteristic peak of imidazole in the prepared catalyst increased strictly and regularly in accordance with the law of embedding weight of 3g, 6g and 12g. The catalyst prepared by impregnating with 6g ionic liquid also showed that characteristic peak intensity of imidazole thereof was higher than that shown by the catalysts obtained by embedding 3g ionic liquid, but lower than that shown by the catalysts obtained by embedding 6g ionic liquid, which was substantially consistent with their catalytic activity.

**Example 14**

Figure 10 showed SEM images of the catalysts obtained by impregnating silica as support with 6g 1-ethyl-3-methyl-imidazolium imidazole salt and the catalysts obtained by embedding 3g, 6g and 12g 1-ethyl-3-methyl-imidazolium imidazole salt in silica as support, respectively.

As shown in Figure 10, Figure a shows an apparent morphology of a strip structure while Figure b shows a regular morphology of silica molecules, the reason of which is that the amount of ionic liquid added is not enough to form a messy composite structure of silane support and ionic liquid. Figures c and d show great changes of apparent morphology of the embedded ionic liquid, and the messy small particles accumulated on the support to form a random composite of silane support and ionic liquid, further illustrating that the ionic liquid was successfully loaded on the support via silane coupling and the cluster phenomenon was more obvious as the loading weight increased. These SEM images fully illustrated that the synthesized ionic liquids by embedding method were distributed in the three-dimensional framework of silica via silane coupling, and the apparent microstructure thereof was significant different from the apparent microstructure of silica.

## Claims

**1.** A method for preparing dimethyl carbonate comprising the following steps:

contacting a raw material comprising ethylene carbonate and methanol with a catalyst to produce dimethyl carbonate;
wherein the catalyst comprises an ionic liquid with an anion and a cation;
wherein the anion and the cation both comprise a nitrogen-containing heterocycle;
wherein the catalyst further comprises a support, and the ionic liquid is embedded in the support in a riveting manner.

**2.** The method for preparing dimethyl carbonate according to claim 1, **characterized in that**,

the reaction is carried out at a reaction temperature ranging from 60°C to 80°C;
a molar ratio of ethylene carbonate to methanol in the raw material comprising ethylene carbonate and methanol is in a range from 1:1 to 1:15, and
the reaction is carried out for a reaction time ranging from 1 minute to 30 h.

**3.** The method for preparing dimethyl carbonate according to claim 1, **characterized in that**, the cation has a structure represented by Formula I or Formula II;

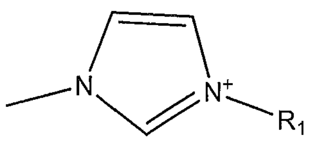 Formula I 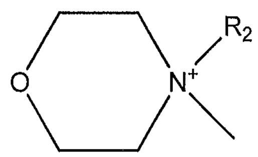 Formula II

the anion has a structure represented by Formula III, Formula IV or Formula V;

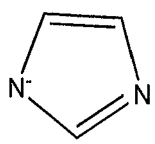 Formula III 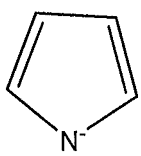 Formula IV 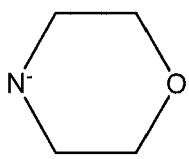 Formula V

wherein $R_1$ and $R_2$ independently from each other are C1-C6 alkyl, C2-C6 alkenyl or C3-C6 aryl.

**4.** The method for preparing dimethyl carbonate according to claim 1, **characterized in that**, the ionic liquid is embedded in the framework of the support, and a weight ratio of the ionic liquid to the support in the catalyst is in a range of 0.01~0.3 : 0.1~0.6.

**5.** The method for preparing dimethyl carbonate according to claim 1, **characterized in that**, the catalyst further comprises a structure regulator, and the ionic liquid is embedded in a composite framework of the structure regulator and the support.

**6.** The method for preparing dimethyl carbonate according to claim 5, **characterized in that**, the structure regulator comprises at least one selected from Mg, Ca, Ba, La, Ce, Zr, Fe, Zn, Li, Cs, and Al, and the weight content of the structure regulator in the catalyst is in a range from 10% to 60%.

**7.** The method for preparing dimethyl carbonate according to claim 1, **characterized in that**, the ionic liquid is prepared by a method comprising the following steps:

a1) adding an alkali into solution I comprising an ionic liquid anion source, and reacting to obtain ionic liquid anion metal salt; and
a2) dissolving the ionic liquid anion metal salt in a solvent, then adding an ionic liquid cation salt, and reacting to obtain the ionic liquid.

**8.** The method for preparing dimethyl carbonate according to claim 7, **characterized in that**,
in step a1), the ionic liquid anion source comprises imidazole, pyrrole or morpholine; in step a2), the solvent comprises a water-carrying agent which is at least one selected from ethanol, benzene, toluene and xylene, and the ionic liquid cation salt is least one selected from 1-$R_1$-3-methylimidazolium bromide, 1-$R_1$-3-methylimidazolium iodide, N-methyl-N-$R_2$-morpholinium bromide, and N-methyl-N-$R_2$-morpholinium iodide salt.

**9.** The method for preparing dimethyl carbonate according to claim 8, **characterized in that**,

in step a1), the concentration of the ionic liquid anion source in the solution I is in a range from 0.05 g/mL to 0.8 g/mL, and a molar ratio of the ionic liquid anion source to the alkali is in a range from 0.9 to 1.1; reaction conditions are as follows: the reaction temperature is in a range from 50°C to 80°C and the reaction time is in a range from 5h to 12 h;
step a1) further comprises: after the reaction is completed, removing the solvent to obtain an imidazole anion salt, a pyrrole anion salt or a morpholine anion salt;
in step a2), reaction conditions are as follows: the reaction temperature is in a range from 25°C to 80°C and the reaction time is in a range from 12h to 48 h.

**10.** The method for preparing dimethyl carbonate according to claim 7, **characterized in that**, a method for preparing the catalyst comprises step b): adding water to a mixture comprising a support precursor and the ionic liquid, and carrying out hydrolysis to obtain the catalyst.

**11.** The method for preparing dimethyl carbonate according to claim 10, **characterized in that**, in step b), the support precursor comprises at least one selected from ethyl orthosilicate, tetrabutyl titanate, aluminum isopropoxide, and sodium aluminate, and a weight ratio of the support precursor, the ionic liquid and water is in a range of 0.2~0.8: 0.03~0.4: 0.1~0.4; and
in step b), the mixture further comprises a solvent, wherein the weight content of the solvent in the mixture is in a range from 10% to 90%.

**12.** The method for preparing dimethyl carbonate according to claim 10, **characterized in that**,

in step b), the hydrolysis is carried out at a hydrolysis temperature ranging from 25°C to 80°C; and
after the hydrolysis, an aging step is carried out to obtain the catalyst, wherein the aging temperature is in a range from 40°C to 120°C and the aging time is in a range from 6h to 24 h.

**13.** The method for preparing dimethyl carbonate according to claim 11, **characterized in that**, in step b), the mixture comprises a structure regulator precursor which comprises at least one selected from corresponding acetate, silicate, hydrochloride, and nitrate of the structure regulator.

**14.** The method for preparing dimethyl carbonate according to claim 13, **characterized in that**, step b) further comprises: mixing the support precursor, the structure regulator precursor with the solvent, adding the ionic liquid, mixing uniformly, and then adding water to carry out hydrolysis to obtain the catalyst, wherein, a weight ratio of the support precursor, the structure regulator precursor, the solvent, the ionic liquid and water is in a range of 0.2~0.8 : 0.05~0.4: 0.3~0.8 : 0.03~0.4: 0.1~0.4.

**Patentansprüche**

1. Verfahren zur Herstellung von Dimethylcarbonat, welches die folgenden Schritte umfasst:

    Inkontaktbringen eines Rohmaterials, das Ethylencarbonat und Methanol umfasst, mit einem Katalysator, um Dimethylcarbonat herzustellen;
    wobei der Katalysator eine ionische Flüssigkeit mit einem Anion und einem Kation umfasst;
    wobei das Anion und das Kation beide einen stickstoffhaltigen Heterocyclus umfassen; wobei der Katalysator ferner einen Träger umfasst und die ionische Flüssigkeit auf nietende Weise in den Träger eingebettet ist.

2. Verfahren zur Herstellung von Dimethylcarbonat nach Anspruch 1, **dadurch gekennzeichnet, dass**

    die Reaktion bei einer Reaktionstemperatur im Bereich von 60 °C bis 80 °C durchgeführt wird;
    ein Molverhältnis von Ethylencarbonat zu Methanol in dem Rohmaterial, das Ethylencarbonat und Methanol umfasst, in einem Bereich von 1:1 bis 1:15 liegt und die Reaktion für eine Reaktionszeit im Bereich von 1 Minute bis 30 h durchgeführt wird.

3. Verfahren zur Herstellung von Dimethylcarbonat nach Anspruch 1, **dadurch gekennzeichnet, dass** das Kation eine Struktur aufweist, die durch Formel I oder Formel II dargestellt ist;

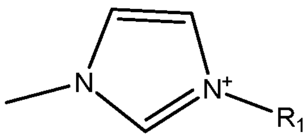 Formel I 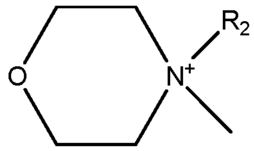 Formel II

das Anion eine Struktur aufweist, die durch Formel III, Formel IV oder Formel V dargestellt ist;

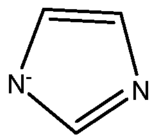 Formel III 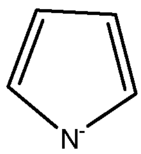 Formel IV 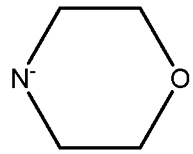 Formel V

wobei $R_1$ und $R_2$ unabhängig voneinander C1-C6-Alkyl, C2-C6-Alkenyl oder C3-C6-Aryl sind.

4. Verfahren zur Herstellung von Dimethylcarbonat nach Anspruch 1, **dadurch gekennzeichnet, dass** die ionische Flüssigkeit in das Gerüst des Trägers eingebettet ist und ein Gewichtsverhältnis der ionischen Flüssigkeit zu dem Träger in dem Katalysator in einem Bereich von 0,01~0,3 : 0,1~0,6 liegt.

5. Verfahren zur Herstellung von Dimethylcarbonat nach Anspruch 1, **dadurch gekennzeichnet, dass** der Katalysator ferner einen Strukturregler umfasst und die ionische Flüssigkeit in ein Verbundgerüst des Strukturreglers und des Trägers eingebettet ist.

6. Verfahren zur Herstellung von Dimethylcarbonat nach Anspruch 5, **dadurch gekennzeichnet, dass** der Strukturregler mindestens eines umfasst, das aus Mg, Ca, Ba, La, Ce, Zr, Fe, Zn, Li, Cs und Al ausgewählt ist, und der Gewichtsgehalt des Strukturreglers in dem Katalysator in einem Bereich von 10% bis 60% liegt.

7. Verfahren zur Herstellung von Dimethylcarbonat nach Anspruch 1, **dadurch gekennzeichnet, dass** die ionische Flüssigkeit durch ein Verfahren hergestellt wird, das die folgenden Schritte umfasst:

    a1) Zugeben eines Alkalis zu einer Lösung I, die eine ionische flüssige Anionenquelle umfasst, und Reagieren, um ein ionisches flüssiges Anionenmetallsalz zu erhalten; und
    a2) Auflösen des ionischen flüssigen Anionenmetallsalzes in einem Lösungsmittel, dann Zugeben eines ionischen flüssigen Kationensalzes und Reagieren, um die ionische Flüssigkeit zu erhalten.

8. Verfahren zur Herstellung von Dimethylcarbonat nach Anspruch 7, **dadurch gekennzeichnet, dass**

in Schritt a1) die ionische flüssige Anionenquelle Imidazol, Pyrrol oder Morpholin umfasst;
in Schritt a2) das Lösungsmittel ein wassertragendes Mittel umfasst, das mindestens eines ist, das aus Ethanol, Benzol, Toluol und Xylol ausgewählt ist, und das ionische flüssige Kationensalz mindestens eines ist, das aus 1-$R_1$-3-Methylimidazoliumbromid, 1-$R_1$-3-Methylimidazoliumiodid, N-Methyl-N-$R_2$-morpholiniumbromid und N-Methyl-N-$R_2$-morpholiniumiodidsalz ausgewählt ist.

**9.** Verfahren zur Herstellung von Dimethylcarbonat nach Anspruch 8, **dadurch gekennzeichnet, dass**

in Schritt a1) die Konzentration der ionischen flüssigen Anionenquelle in der Lösung I in einem Bereich von 0,05 g/ml bis 0,8 g/ml liegt und ein Molverhältnis der ionischen flüssigen Anionenquelle zu dem Alkali in einem Bereich von 0,9 bis 1,1 liegt; die Reaktionsbedingungen wie folgt sind: die Reaktionstemperatur liegt in einem Bereich von 50 °C bis 80 °C und die Reaktionszeit liegt in einem Bereich von 5 h bis 12 h; Schritt a1) ferner umfasst: nachdem die Reaktion abgeschlossen ist, Entfernen des Lösungsmittels, um ein Imidazolanionensalz, ein Pyrrolanionensalz oder ein Morpholinanionensalz zu erhalten;
in Schritt a2) die Reaktionsbedingungen wie folgt sind: die Reaktionstemperatur liegt in einem Bereich von 25 °C bis 80 °C und die Reaktionszeit liegt in einem Bereich von 12 h bis 48 h.

**10.** Verfahren zur Herstellung von Dimethylcarbonat nach Anspruch 7, **dadurch gekennzeichnet, dass** ein Verfahren zur Herstellung des Katalysators Schritt b) umfasst: Zugeben von Wasser zu einer Mischung, die einen Trägervorläufer und die ionische Flüssigkeit umfasst, und Durchführen einer Hydrolyse, um den Katalysator zu erhalten.

**11.** Verfahren zur Herstellung von Dimethylcarbonat nach Anspruch 10, **dadurch gekennzeichnet, dass** in Schritt b) der Trägervorläufer mindestens eines umfasst, das aus Ethylorthosilicat, Tetrabutyltitanat, Aluminiumisopropoxid und Natriumaluminat ausgewählt ist, und ein Gewichtsverhältnis des Trägervorläufers, der ionischen Flüssigkeit und Wasser in einem Bereich von 0,2~0,8 : 0,03~0,4 : 0,1~0,4 liegt; und in Schritt b) die Mischung ferner ein Lösungsmittel umfasst, wobei der Gewichtsgehalt des Lösungsmittels in der Mischung in einem Bereich von 10 % bis 90 % liegt.

**12.** Verfahren zur Herstellung von Dimethylcarbonat nach Anspruch 10, **dadurch gekennzeichnet, dass**

in Schritt b) die Hydrolyse bei einer Hydrolysetemperatur im Bereich von 25 °C bis 80 °C durchgeführt wird; und nach der Hydrolyse ein Alterungsschritt durchgeführt wird, um den Katalysator zu erhalten, wobei die Alterungstemperatur in einem Bereich von 40 °C bis 120 °C liegt und die Alterungszeit in einem Bereich von 6 h bis 24 h liegt.

**13.** Verfahren zur Herstellung von Dimethylcarbonat nach Anspruch 11, **dadurch gekennzeichnet, dass** in Schritt b) die Mischung einen Strukturreglervorläufer umfasst, der mindestens eines umfasst, das aus entsprechendem Acetat, Silicat, Hydrochlorid und Nitrat des Strukturreglers ausgewählt ist.

**14.** Verfahren zur Herstellung von Dimethylcarbonat nach Anspruch 13, **dadurch gekennzeichnet, dass** Schritt b) ferner umfasst: Mischen des Trägervorläufers, des Strukturreglervorläufers mit dem Lösungsmittel, Zugeben der ionischen Flüssigkeit, gleichmäßiges Mischen und dann Zugeben von Wasser, um eine Hydrolyse durchzuführen, um den Katalysator zu erhalten, wobei ein Gewichtsverhältnis des Trägervorläufers, des Strukturreglervorläufers, des Lösungsmittels, der ionischen Flüssigkeit und Wasser in einem Bereich von 0,2~0,8 : 0,05~0,4 : 0,3~0,8 : 0,03~0,4 : 0,1~0,4 liegt.

## Revendications

**1.** Procédé de préparation de carbonate de diméthyle comprenant les étapes suivantes :

la mise en contact d’une matière première comprenant du carbonate d’éthylène et du méthanol avec un catalyseur pour produire du carbonate de diméthyle ;
dans lequel le catalyseur comprend un liquide ionique avec un anion et un cation ;
dans lequel l’anion et le cation comprennent tous deux un hétérocycle contenant de l’azote ;
dans lequel le catalyseur comprend en outre un support, et le liquide ionique est noyé dans le support d’une manière rivetée.

**2.** Procédé de préparation de carbonate de diméthyle selon la revendication 1, **caractérisé en ce que**

la réaction est réalisée à une température de réaction allant de 60 °C à 80 °C ;
un rapport molaire du carbonate d'éthylène au méthanol dans la matière première comprenant du carbonate d'éthylène et du méthanol est dans une plage allant de 1:1 à 1:15, et
la réaction est réalisée pendant un temps de réaction allant de 1 minute à 30 h.

**3.** Procédé de préparation de carbonate de diméthyle selon la revendication 1, **caractérisé en ce que** le cation a une structure représentée par la Formule I ou la Formule II ;

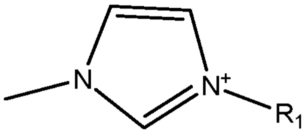 Formule I 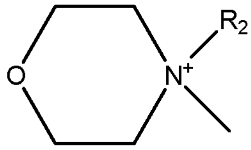 Formule II

l'anion a une structure représentée par la Formule III, Formule IV ou Formule V;

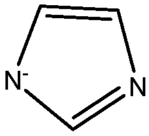 Formule III 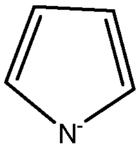 Formule IV 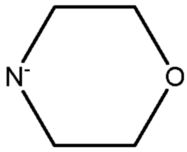 Formule V

dans laquelle $R_1$ et $R_2$, indépendamment l'un de l'autre, sont un alkyle en C1 à C6, un alcényle en C2 à C6 ou un aryle en C3 à C6.

**4.** Procédé de préparation de carbonate de diméthyle selon la revendication 1, **caractérisé en ce que** le liquide ionique est noyé dans la charpente du support, et un rapport en poids du liquide ionique au support dans le catalyseur est dans une plage de 0,01 ~ 0,3 : 0,1 ~ 0,6.

**5.** Procédé de préparation de carbonate de diméthyle selon la revendication 1, **caractérisé en ce que** le catalyseur comprend en outre un régulateur de structure, et le liquide ionique est noyé dans une charpente composite du régulateur de structure et du support.

**6.** Procédé de préparation de carbonate de diméthyle selon la revendication 5, **caractérisé en ce que** le régulateur de structure comprend au moins l'un choisi parmi Mg, Ca, Ba, La, Ce, Zr, Fe, Zn, Li, Cs, et Al, et la teneur en poids du régulateur de structure dans le catalyseur est dans une plage de 10 % à 60 %.

**7.** Procédé de préparation de carbonate de diméthyle selon la revendication 1, **caractérisé en ce que** le liquide ionique est préparé par un procédé comprenant les étapes suivantes :

a1) l'ajout d'un alcali dans une solution I comprenant une source d'anion liquide ionique, et la réaction pour obtenir un sel métallique d'anion liquide ionique ; et
a2) la dissolution du sel métallique d'anion liquide ionique dans un solvant, puis l'ajout d'un sel de cation liquide ionique, et la réaction pour obtenir le liquide ionique.

**8.** Procédé de préparation de carbonate de diméthyle selon la revendication 7, **caractérisé en ce que**

dans l'étape a1), la source d'anion liquide ionique comprend de l'imidazole, du pyrrole ou de la morpholine ;
dans l'étape a2), le solvant comprend un agent porteur d'eau qui est au moins l'un choisi parmi l'éthanol, le benzène, le toluène et le xylène, et le sel de cation liquide ionique est au moins l'un choisi parmi le bromure de 1-$R_1$-3-méthylimidazolium, l'iodure de 1-$R_1$-3-méthylimidazolium, le bromure de N-méthyl-N-$R_2$-morpholinium, et le sel d'iodure de N-méthyl-N-$R_2$-morpholinium.

**9.** Procédé de préparation de carbonate de diméthyle selon la revendication 8, **caractérisé en ce que**

dans l'étape a1), la concentration de la source d'anion liquide ionique dans la solution I est dans une plage de 0,05 g/ml à 0,8 g/ml, et un rapport molaire de la source d'anion liquide ionique à l'alcali est dans une plage de 0,9 à 1,1 ;

les conditions de réaction sont les suivantes : la température de réaction est dans une plage de 50 °C à 80 °C et le temps de réaction est dans une plage de 5 h à 12 h ;
l'étape a1) comprend en outre : après que la réaction est terminée, l'élimination du solvant pour obtenir un sel d'anion imidazole, un sel d'anion pyrrole ou un sel d'anion morpholine ;
dans l'étape a2), les conditions de réaction sont les suivantes : la température de réaction est dans une plage de 25 °C à 80 °C et le temps de réaction est dans une plage de 12 h à 48 h.

**10.** Procédé de préparation de carbonate de diméthyle selon la revendication 7, **caractérisé en ce que** un procédé de préparation du catalyseur comprend l'étape b) : l'ajout d'eau à un mélange comprenant un précurseur de support et le liquide ionique, et la réalisation d'une hydrolyse pour obtenir le catalyseur.

**11.** Procédé de préparation de carbonate de diméthyle selon la revendication 10, **caractérisé en ce que** dans l'étape b), le précurseur de support comprend au moins l'un choisi parmi l'orthosilicate d'éthyle, le titanate de tétrabutyle, l'isopropoxyde d'aluminium, et l'aluminate de sodium, et un rapport en poids du précurseur de support, du liquide ionique et de l'eau est dans une plage de 0,2 ~ 0,8 : 0,03 ~ 0,4 : 0,1 ~ 0,4 ; et dans l'étape b), le mélange comprend en outre un solvant, dans lequel la teneur en poids du solvant dans le mélange est dans une plage de 10 % à 90 %.

**12.** Procédé de préparation de carbonate de diméthyle selon la revendication 10, **caractérisé en ce que**

dans l'étape b), l'hydrolyse est réalisée à une température d'hydrolyse allant de 25 °C à 80 °C ; et
après l'hydrolyse, une étape de vieillissement est réalisée pour obtenir le catalyseur, dans lequel la température de vieillissement est dans une plage de 40 °C à 120 °C et le temps de vieillissement est dans une plage de 6 h à 24 h.

**13.** Procédé de préparation de carbonate de diméthyle selon la revendication 11, **caractérisé en ce que** dans l'étape b), le mélange comprend un précurseur de régulateur de structure qui comprend au moins l'un choisi parmi l'acétate, le silicate, le chlorhydrate, et le nitrate correspondants du régulateur de structure.

**14.** Procédé de préparation de carbonate de diméthyle selon la revendication 13, **caractérisé en ce que** l'étape b) comprend en outre : le mélange du précurseur de support, du précurseur de régulateur de structure avec le solvant, l'ajout du liquide ionique, le mélange uniformément, et ensuite l'ajout d'eau pour réaliser une hydrolyse pour obtenir le catalyseur, dans lequel, un rapport en poids du précurseur de support, du précurseur de régulateur de structure, du solvant, du liquide ionique et de l'eau est dans une plage de 0,2 ~ 0,8 : 0,05 ~ 0,4 : 0,3 ~ 0,8 : 0,03 ~ 0,4 : 0,1 ~ 0,4.

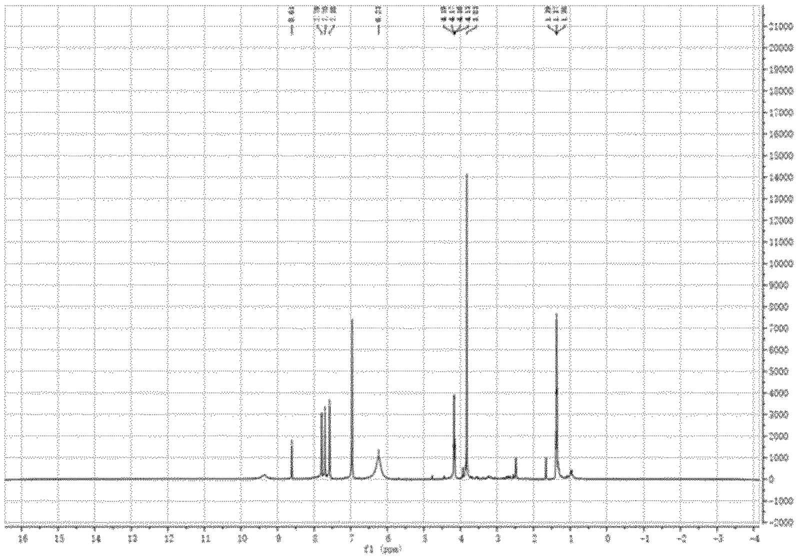

FIG.1

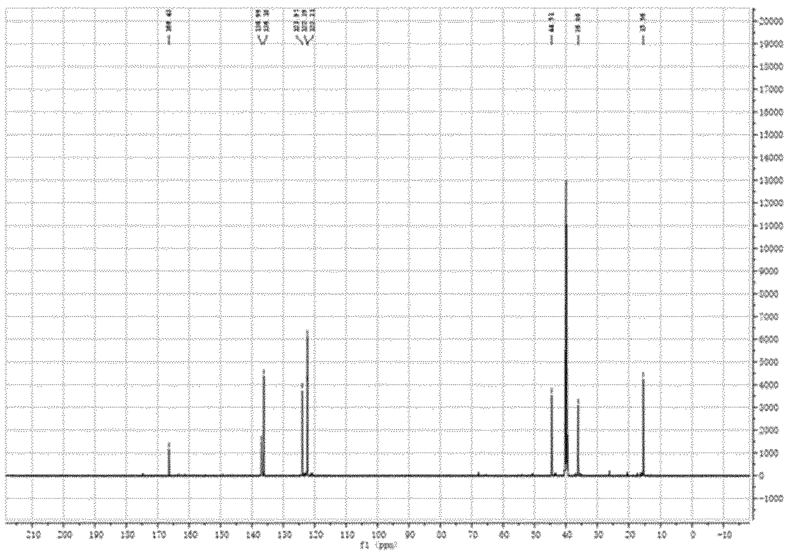

FIG.2

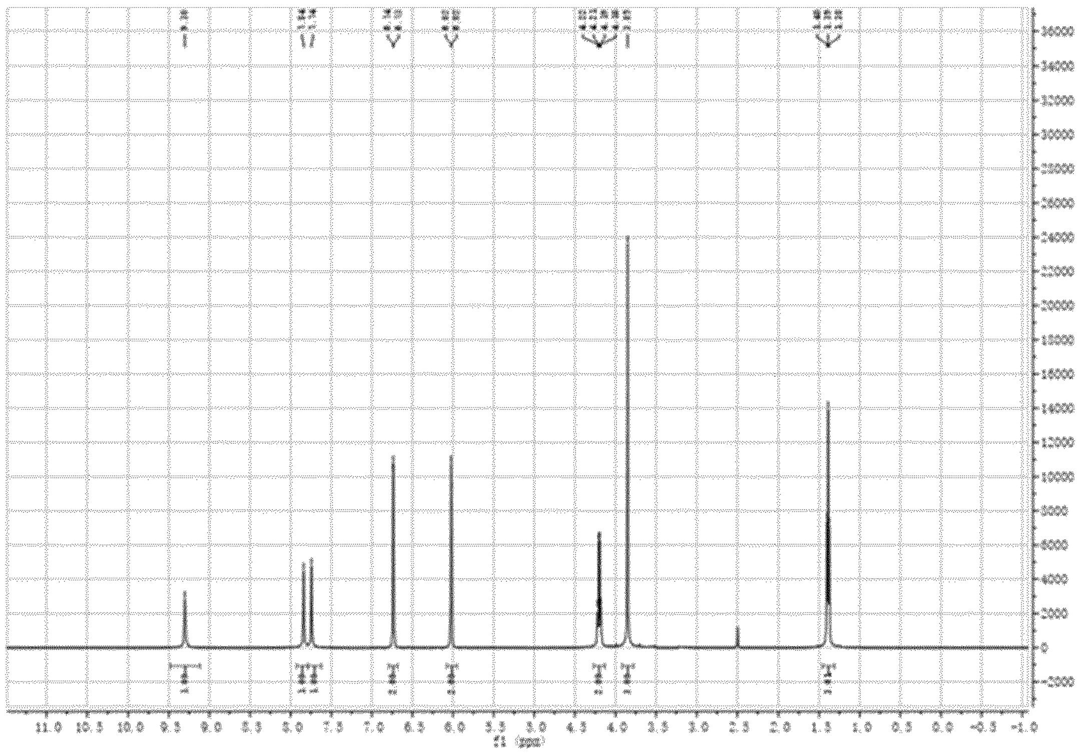

FIG.3

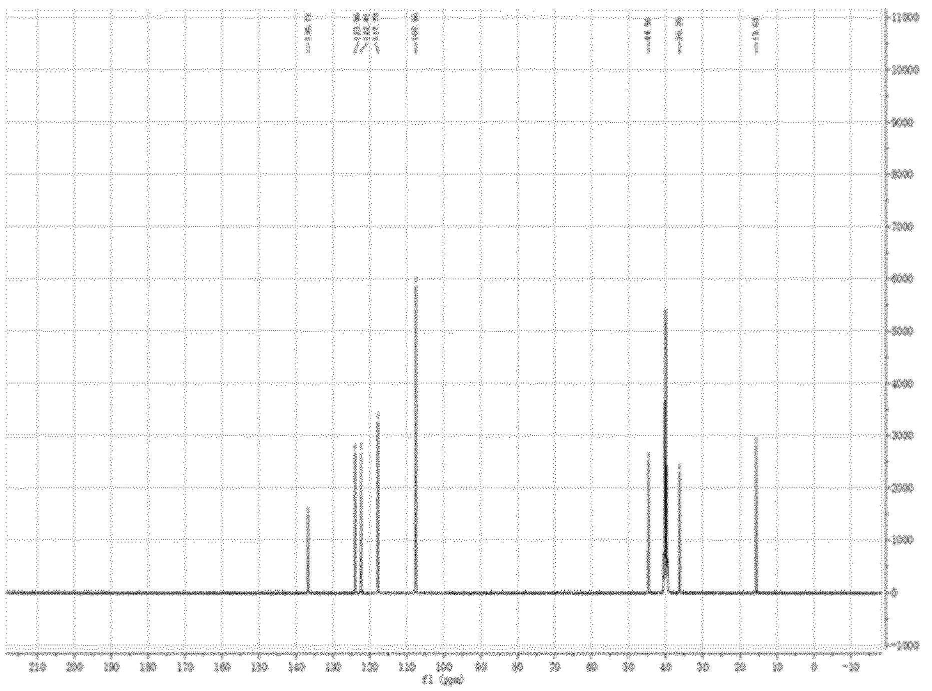

FIG.4

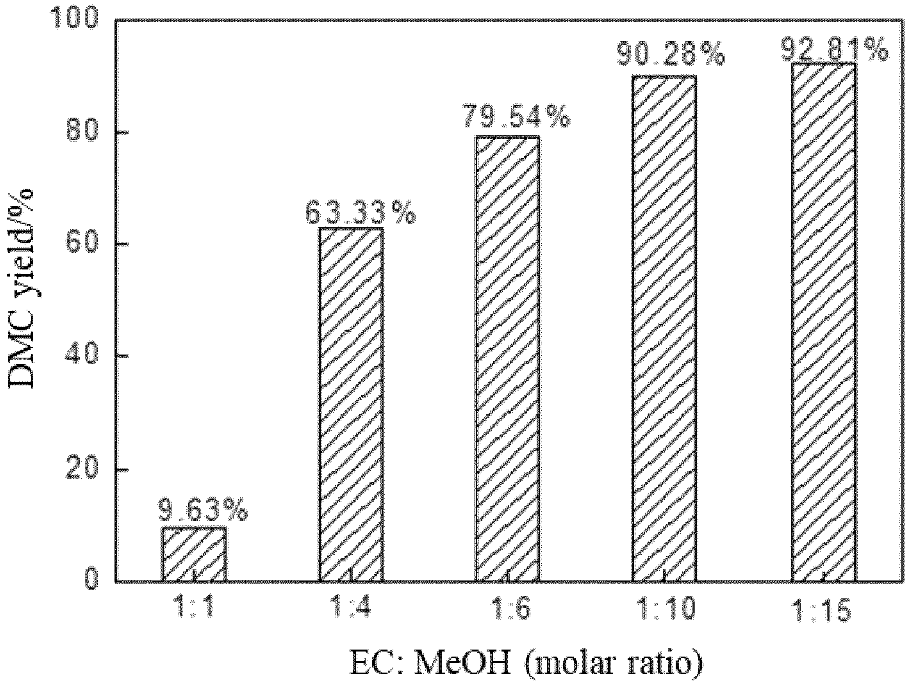
DMC yield/%
100
80
60
40
20
0
9.63%
63.33%
79.54%
90.28%
92.81%
1:1
1:4
1:6
1:10
1:15
EC: MeOH (molar ratio)

**FIG.5**

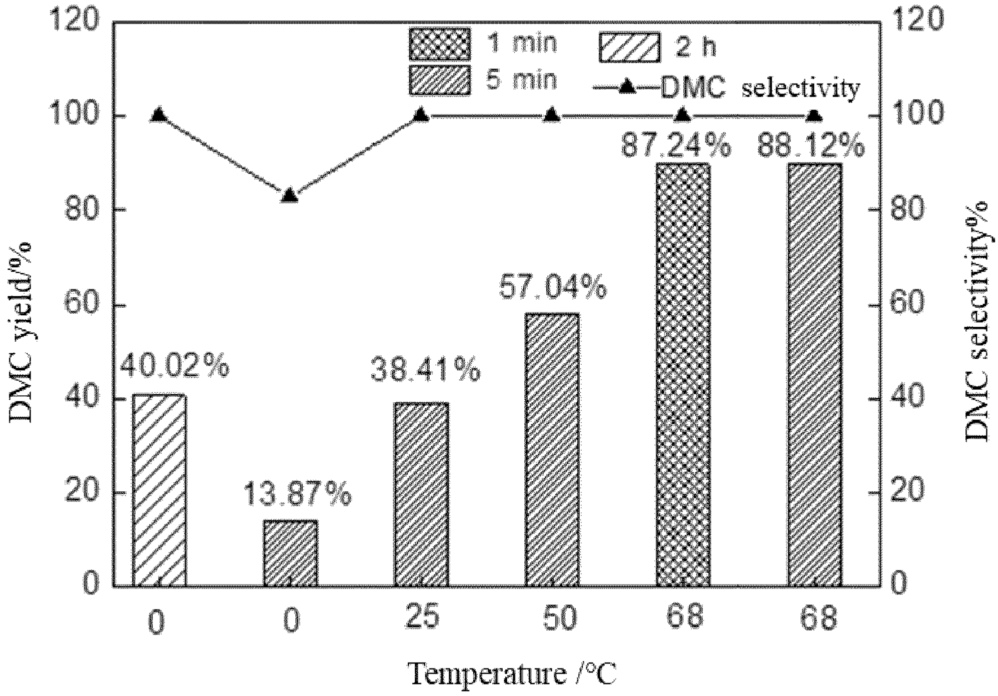
1 min
2 h
5 min
DMC selectivity
DMC yield/%
DMC selectivity%
120
100
80
60
40
20
0
40.02%
13.87%
38.41%
57.04%
87.24%
88.12%
0
0
25
50
68
68
Temperature /°C

**FIG.6**

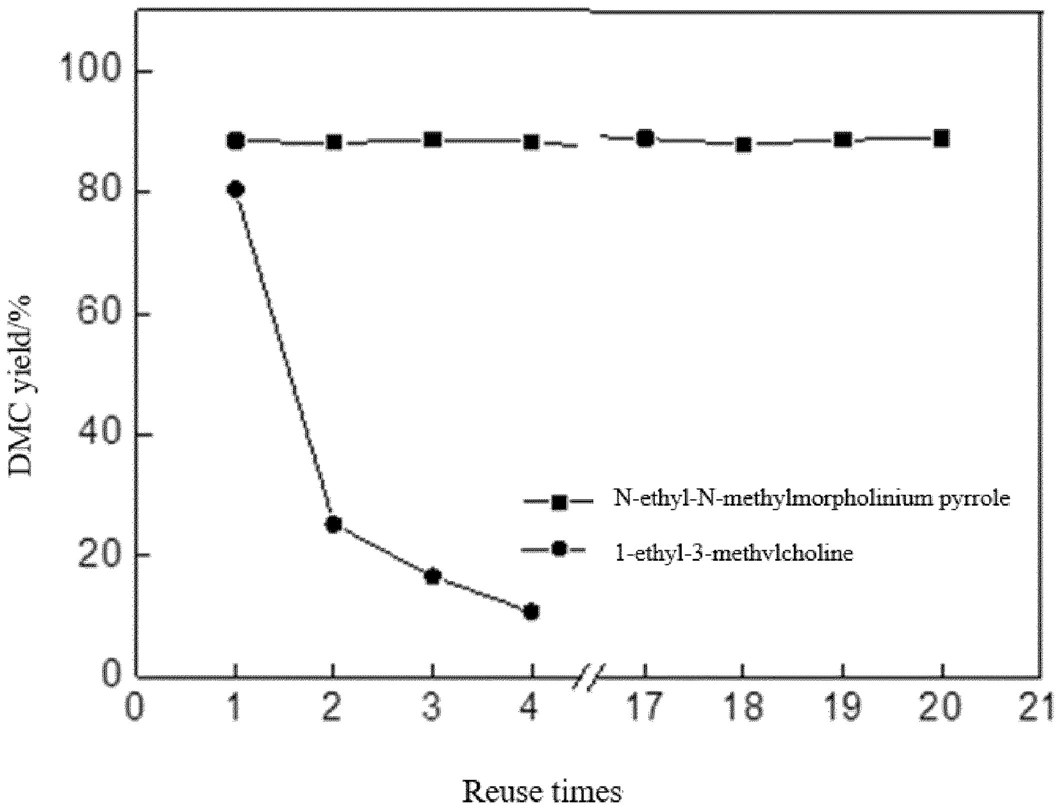
100
80
60
40
20
0
DMC yield/%
0
1
2
3
4
17
18
19
20
21
N-ethyl-N-methylmorpholinium pyrrole
1-ethyl-3-methvlcholine
Reuse times

FIG.7

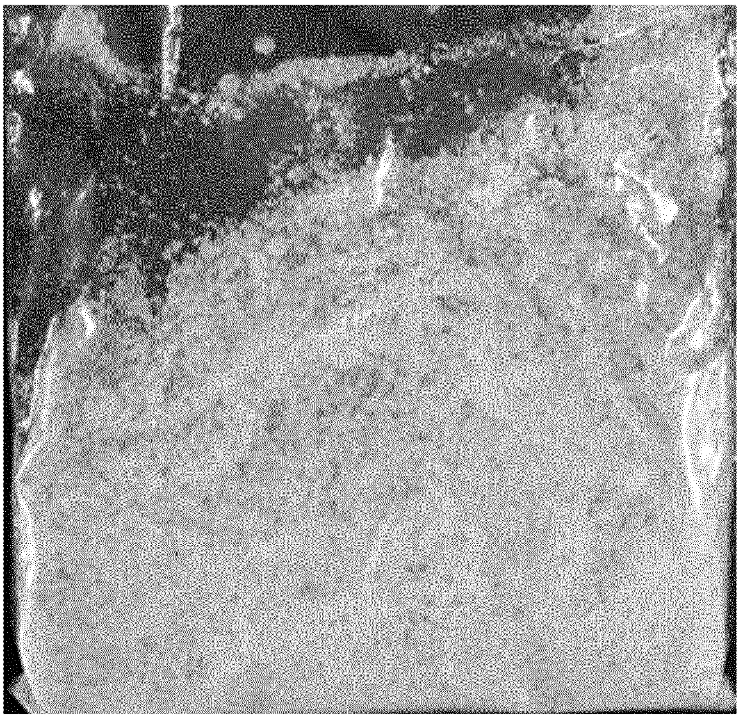

FIG.8

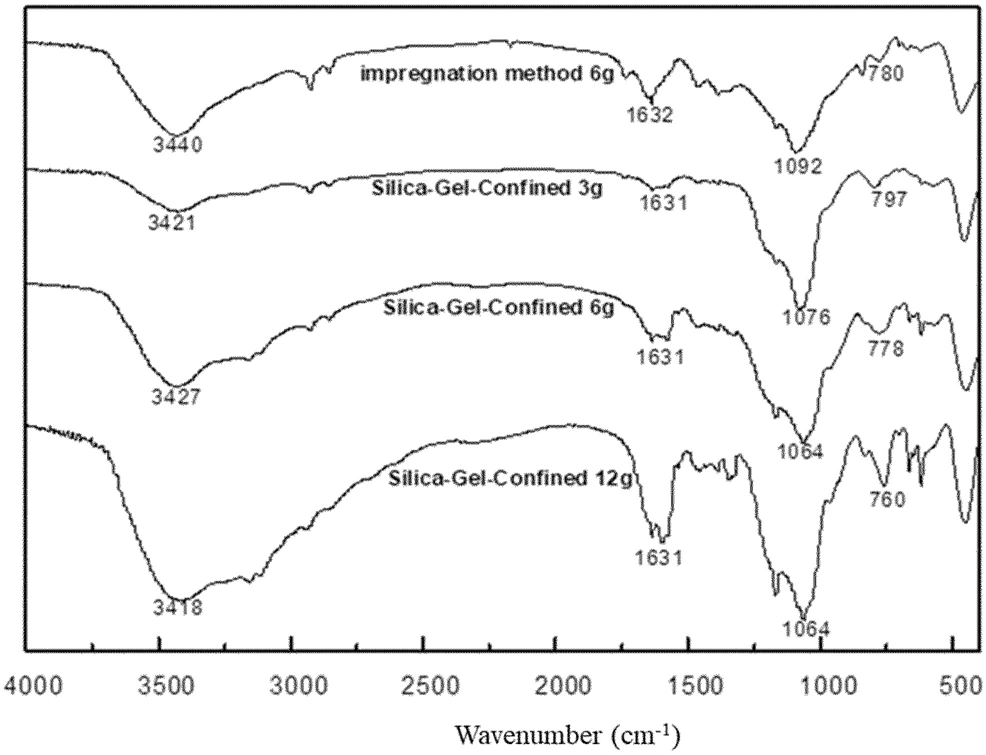
impregnation method 6g
3440
1632
1092
780
Silica-Gel-Confined 3g
3421
1631
1076
797
Silica-Gel-Confined 6g
3427
1631
1064
778
Silica-Gel-Confined 12g
3418
1631
1064
760
4000
3500
3000
2500
2000
1500
1000
500
Wavenumber ($cm^{-1}$)

FIG.9

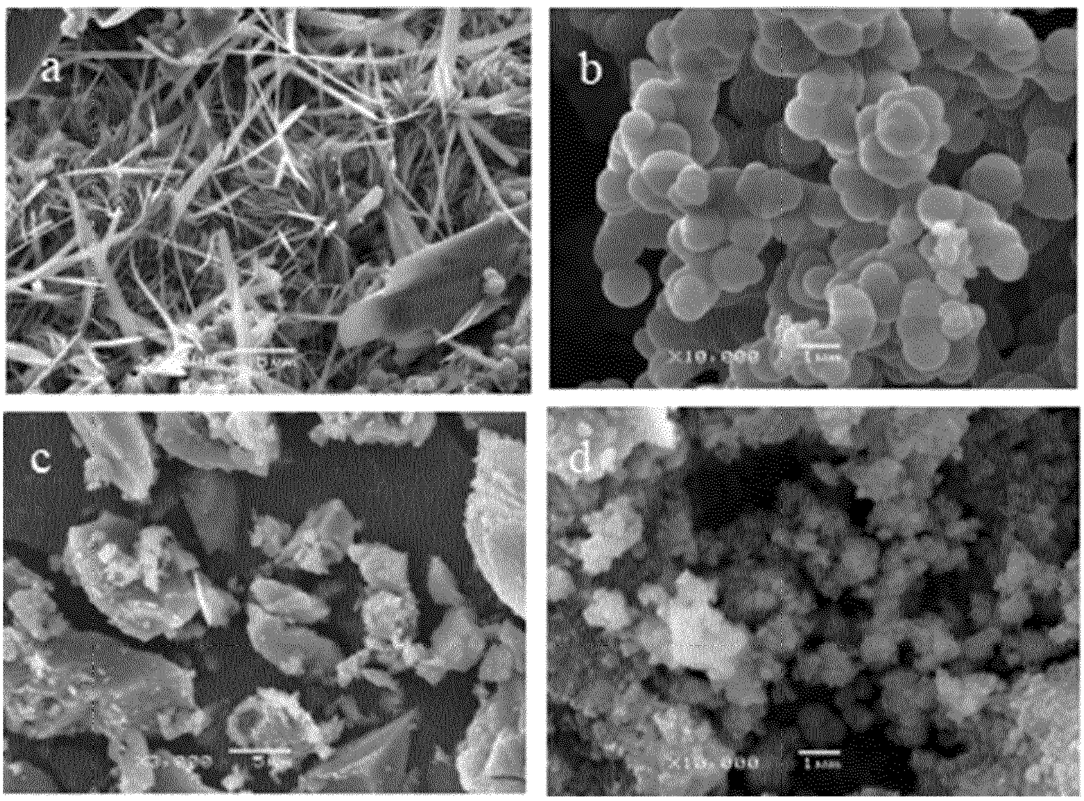
a
b
c
d

FIG.10

## REFERENCES CITED IN THE DESCRIPTION

*This list of references cited by the applicant is for the reader’s convenience only. It does not form part of the European patent document. Even though great care has been taken in compiling the references, errors or omissions cannot be excluded and the EPO disclaims all liability in this regard.*

### Patent documents cited in the description

- CN 109704968 A
- CN 103641720 A